# EUROPEAN PATENT APPLICATION

(11) **EP 4 190 901 A1**
(43) Date of publication of application: **07.06.2023**
(21) Application number: 21851121.0
(22) Date of filing: 21.07.2021
(51) Int. Cl.: C12N 15/12, A01K 67/027, A01K 67/033, C07K 14/705, C12N 1/15, C12N 1/19, C12N 1/21, C12N 5/10, C12N 15/09, G01N 21/64

(54) **NITRO COMPOUND DETECTION ELEMENT**

(30) Priority: 27.07.2020 JP 2020126686
(71) Applicant: SUMITOMO CHEMICAL COMPANY, LIMITED, Tokyo 103-6020 (JP)
(72) Inventor: OZOE, Atsufumi, Osaka-shi, Osaka 554-8558 (JP); TAKAHASHI, Yasuhiko, Osaka-shi, Osaka 554-8558 (JP)
(74) Representative: Vossius & Partner Patentanwälte Rechtsanwälte mbB
(86) International application number: PCT/JP2021/027267
(87) International publication number: WO 2022/024903

(57) **Abstract**

An object is to provide a technique for detecting a nitro compound. The object is achieved by a nitro compound detection element formed from a specific type of an insect olfactory receptor or from an insect olfactory receptor having a specific mutation introduced.

## Description

### Technical Field

The present disclosure relates to a nitro compound detection element.

### Background Art

Many of the nitro compounds are explosive, and there is demand for the development of detection technology for these compounds. Animals such as dogs are known to recognize nitro compounds, and olfactory receptor genes of mammals (mice and rats) have been identified as biological factors involved in this recognition (NPL 1).

The insects have been shown to have olfactory functionality of highly specific odorant recognition, and its use in olfactory sensors and other applications is being explored. The olfactory organs of insects contain an olfactory receptor complex formed from an olfactory receptor that recognizes odors and an olfactory receptor co-receptor. Upon activation by an odorant, the olfactory receptor complex shows ion channel activity, and the odorant is thus detected. However, it was unknown that such insect olfactory receptors can detect nitro compounds.

### Citation List

### Patent Literature

PTL 1: JP2013-27376A
PTL 2: JP2018-59786A
PTL 3: JP2012-78351A

### Non-patent Literature

NPL 1: Li J, Haddad R, Chen S, Santos V, Luetje CW. A broadly tuned mouse odorant receptor that detects nitrotoluenes. J Neurochem. 2012 Jun; 121 (6): 881-890.

### Summary of Invention

### Technical Problem

An object of the present disclosure is to provide a technique for detecting a nitro compound.

### Solution to Problem

The present inventors conducted extensive research to achieve the object and found that a specific type of insect olfactory receptor or an insect olfactory receptor having a specific mutation introduced has nitro compound responsiveness. The inventors conducted further research based on this finding and completed the invention in the present disclosure. Specifically, the present disclosure includes the following aspects.
Item 1. A nitro compound detection element comprising an olfactory receptor protein, the olfactory receptor protein comprising
   (1) amino acid sequence 1C composed of amino acid sequence 1A of SEQ ID NO: 1 or amino acid sequence 1B with at least 80% identity to the amino acid sequence 1A, the amino acid sequence 1A and the amino acid sequence 1B each containing a mutation, wherein the amino acid sequence 1C contains mutation 1a, and the mutation 1a is deletion of a partial or entire region of the first extracellular loop;
   (2) amino acid sequence 2C composed of amino acid sequence 2A of SEQ ID NO: 2 or amino acid sequence 2B with at least 80% identity to the amino acid sequence 2A, the amino acid sequence 2A and the amino acid sequence 2B each containing a mutation,
      wherein the amino acid sequence 2C contains mutation 2a, and the mutation 2a is substitution 2ax or substitution 2ay,
      the substitution 2ax is substitution of at least one amino acid selected from the group consisting of L60, M138, and A152 in the amino acid sequence 2A, and
      the substitution 2ay corresponds to the substitution 2ax in the amino acid sequence 2B;
   (3) amino acid sequence 3C composed of amino acid sequence 3A of SEQ ID NO: 3 or amino acid sequence 3B with at least 80% identity to the amino acid sequence 3A, the amino acid sequence 3A and the amino acid sequence 3B each containing a mutation,
      wherein the amino acid sequence 3C contains mutation 3a and mutation 3b,
      the mutation 3a is substitution 3ax or substitution 3ay,
      the substitution 3ax is substitution of amino acids S114, A120, H162, V186, V352, and S368 in the amino acid sequence 3A, and the substitution 3ay corresponds to the substitution 3ax in the amino acid sequence 3B,
      the mutation 3b is substitution of a charged amino acid into an uncharged amino acid in a region from the third extracellular loop to the third intracellular loop;
   (4) amino acid sequence 4C composed of amino acid sequence 4A of SEQ ID NO: 4 or amino acid sequence 4B with at least 80% identity to the amino acid sequence 4A, the amino acid sequence 4A and the amino acid sequence 4B each containing a mutation,
      wherein the amino acid sequence 4C contains mutation 4a, and the mutation 4a is substitution 4ax or substitution 4ay,
      the substitution 4ax is substitution of amino acids M61, C104, R112, G181, V189, and 1327 in the amino acid sequence 4A, and the substitution 4ay corresponds to the substitution 4ax in the amino acid sequence 4B; or
   (5) amino acid sequence 5C composed of amino acid sequence 5A of SEQ ID NO: 5 or amino acid sequence 5B with at least 80% identity to the amino acid sequence 5A, the amino acid sequence 5A and the amino acid sequence 5B each containing a mutation,
      wherein the amino acid sequence 5C contains mutation 5a, and the mutation 5a is substitution 5ax or substitution 5ay,
      the substitution 5ax is substitution of amino acid L178 in the amino acid sequence 5A, and the substitution 5ay corresponds to the substitution 5ax in the amino acid sequence 5B.
Item 1A. Use of an olfactory receptor protein in a nitro compound detection element, the olfactory receptor protein comprising
   (1) amino acid sequence 1C composed of amino acid sequence 1A of SEQ ID NO: 1 or amino acid sequence 1B with at least 80% identity to the amino acid sequence 1A, the amino acid sequence 1A and the amino acid sequence 1B each containing a mutation, wherein the amino acid sequence 1C contains mutation 1a, and the mutation 1a is deletion of a partial or entire region of the first extracellular loop;
   (2) amino acid sequence 2C composed of amino acid sequence 2A of SEQ ID NO: 2 or amino acid sequence 2B with at least 80% identity to the amino acid sequence 2A, the amino acid sequence 2A and the amino acid sequence 2B each containing a mutation,
      wherein the amino acid sequence 2C contains mutation 2a, and the mutation 2a is substitution 2ax or substitution 2ay,
      the substitution 2ax is substitution of at least one amino acid selected from the group consisting of L60, M138, and A152 in the amino acid sequence 2A, and
      the substitution 2ay corresponds to the substitution 2ax in the amino acid sequence 2B;
   (3) amino acid sequence 3C composed of amino acid sequence 3A of SEQ ID NO: 3 or amino acid sequence 3B with at least 80% identity to the amino acid sequence 3A, the amino acid sequence 3A and the amino acid sequence 3B each containing a mutation,
      wherein the amino acid sequence 3C contains mutation 3a and mutation 3b,
      the mutation 3a is substitution 3ax or substitution 3ay,
      the substitution 3ax is substitution of amino acids S114, A120, H162, V186, V352, and S368 in the amino acid sequence 3A, and the substitution 3ay corresponds to the substitution 3ax in the amino acid sequence 3B,
      the mutation 3b is substitution of a charged amino acid into an uncharged amino acid in a region from the third extracellular loop to the third intracellular loop;
   (4) amino acid sequence 4C composed of amino acid sequence 4A of SEQ ID NO: 4 or amino acid sequence 4B with at least 80% identity to the amino acid sequence 4A, the amino acid sequence 4A and the amino acid sequence 4B each containing a mutation,
      wherein the amino acid sequence 4C contains mutation 4a, and the mutation 4a is substitution 4ax or substitution 4ay,
      the substitution 4ax is substitution of amino acids M61, C104, R112, G181, V189, and I327 in the amino acid sequence 4A, and the substitution 4ay corresponds to the substitution 4ax in the amino acid sequence 4B; or
   (5) amino acid sequence 5C composed of amino acid sequence 5A of SEQ ID NO: 5 or amino acid sequence 5B with at least 80% identity to the amino acid sequence 5A, the amino acid sequence 5A and the amino acid sequence 5B each containing a mutation,
      wherein the amino acid sequence 5C contains mutation 5a, and the mutation 5a is substitution 5ax or substitution 5ay,
      the substitution 5ax is substitution of amino acid L178 in the amino acid sequence 5A, and the substitution 5ay corresponds to the substitution 5ax in the amino acid sequence 5B.
Item 1B. Use of an olfactory receptor protein in the production of a nitro compound detection element, the olfactory receptor protein comprising
   (1) amino acid sequence 1C composed of amino acid sequence 1A of SEQ ID NO: 1 or amino acid sequence 1B with at least 80% identity to the amino acid sequence 1A, the amino acid sequence 1A and the amino acid sequence 1B each containing a mutation, wherein the amino acid sequence 1C contains mutation 1a, and the mutation 1a is deletion of a partial or entire region of the first extracellular loop;
   (2) amino acid sequence 2C composed of amino acid sequence 2A of SEQ ID NO: 2 or amino acid sequence 2B with at least 80% identity to the amino acid sequence 2A, the amino acid sequence 2A and the amino acid sequence 2B each containing a mutation,
      wherein the amino acid sequence 2C contains mutation 2a, and the mutation 2a is substitution 2ax or substitution 2ay,
      the substitution 2ax is substitution of at least one amino acid selected from the group consisting of L60, M138, and A152 in the amino acid sequence 2A, and
      the substitution 2ay corresponds to the substitution 2ax in the amino acid sequence 2B;
   (3) amino acid sequence 3C composed of amino acid sequence 3A of SEQ ID NO: 3 or amino acid sequence 3B with at least 80% identity to the amino acid sequence 3A, the amino acid sequence 3A and the amino acid sequence 3B each containing a mutation,
      wherein the amino acid sequence 3C contains mutation 3a and mutation 3b,
      the mutation 3a is substitution 3ax or substitution 3ay,
      the substitution 3ax is substitution of amino acids S114, A120, H162, V186, V352, and S368 in the amino acid sequence 3A, and the substitution 3ay corresponds to the substitution 3ax in the amino acid sequence 3B,
      the mutation 3b is substitution of a charged amino acid into an uncharged amino acid in a region from the third extracellular loop to the third intracellular loop;
   (4) amino acid sequence 4C composed of amino acid sequence 4A of SEQ ID NO: 4 or amino acid sequence 4B with at least 80% identity to the amino acid sequence 4A, the amino acid sequence 4A and the amino acid sequence 4B each containing a mutation,
      wherein the amino acid sequence 4C contains mutation 4a, and the mutation 4a is substitution 4ax or substitution 4ay,
      the substitution 4ax is substitution of amino acids M61, C104, R112, G181, V189, and I327 in the amino acid sequence 4A, and the substitution 4ay corresponds to the substitution 4ax in the amino acid sequence 4B; or
   (5) amino acid sequence 5C composed of amino acid sequence 5A of SEQ ID NO: 5 or amino acid sequence 5B with at least 80% identity to the amino acid sequence 5A, the amino acid sequence 5A and the amino acid sequence 5B each containing a mutation,
      wherein the amino acid sequence 5C contains mutation 5a, and the mutation 5a is substitution 5ax or substitution 5ay,
      the substitution 5ax is substitution of amino acid L178 in the amino acid sequence 5A, and the substitution 5ay corresponds to the substitution 5ax in the amino acid sequence 5B.
Item 2. The nitro compound detection element according to Item 1, wherein
   in the mutation 1a, the deleted region is a region of 5 to 30 amino acids from the amino acid at the N-terminus;
   in the mutation 2a, the amino acid after mutation of L60 is proline, the amino acid after mutation of M138 is a branched-chain amino acid, and/or the amino acid after mutation of A152 is a hydrophilic neutral amino acid;
   in the mutation 3a, the amino acid after mutation of S114 is an aliphatic amino acid, the amino acid after mutation of A120 is an acidic amino acid, the amino acid after mutation of H162 is an aromatic amino acid, the amino acid after mutation of V186 is an aliphatic amino acid, the amino acid after mutation of V352 is a sulfur-containing amino acid, and the amino acid after mutation of S368 is proline;
   in the mutation 3b, the number of substitutions of a charged amino acid into an uncharged amino acid is 6 to 18;
   in the mutation 4a, the amino acid after mutation of M61 is a hydrophilic neutral amino acid, the amino acid after mutation of C104 is a basic amino acid, the amino acid after mutation of R112 is a hydrophilic neutral amino acid, the amino acid after mutation of G181 is an aliphatic amino acid, the amino acid after mutation of V189 is a branched-chain amino acid, and the amino acid after mutation of I327 is a branched-chain amino acid; and
   in the mutation 5a, the amino acid after mutation of L178 is proline.
Item 3. The nitro compound detection element according to Item 1 or 2, wherein
   in the mutation 1a, the deleted region is a region of 15 to 25 amino acids from the amino acid at the N-terminus;
   in the mutation 2a, the amino acid after mutation of L60 is proline, the amino acid after mutation of M138 is isoleucine, and/or the amino acid after mutation of A152 is threonine;
   in the mutation 3a, the amino acid after mutation of S114 is alanine, the amino acid after mutation of A120 is glutamic acid, the amino acid after mutation of H162 is tyrosine, the amino acid after mutation of V186 is alanine, the amino acid after mutation of V352 is methionine, and the amino acid after mutation of S368 is proline;
   in the mutation 3b, the number of substitutions of a charged amino acid into an uncharged amino acid is 8 to 14;
   in the mutation 4a, the amino acid after mutation of M61 is threonine, the amino acid after mutation of C104 is arginine, the amino acid after mutation of R112 is glutamine, the amino acid after mutation of G181 is alanine, the amino acid after mutation of V189 is isoleucine, and the amino acid after mutation of I327 is valine; and
   in the mutation 5a, the amino acid after mutation of L178 is proline.
Item 4. The nitro compound detection element according to any one of Items 1 to 3, wherein the nitro compound is a compound with a structure such that a nitro group is directly linked to a benzene ring and/or a compound having two or more nitro groups.
Item 5. The nitro compound detection element according to any one of Items 1 to 4,
   wherein the olfactory receptor protein contains amino acid sequence B composed of amino acid sequence A containing a mutation,
   the amino acid sequence A is in a corresponding wild-type olfactory receptor protein and represented by formula (1): φ₇X₁X₂Z₁X₃X₄Z₂Z₃φ₆Uφ₅φ₄φ₃φ₂φ₁X₅X₆ wherein X₁ to X₆ and Z₁ to Z₃ are amino acids derived from the amino acid sequence of a wild-type insect olfactory receptor, φ₁ represents a hydrophobic amino acid, φ₂ to φ₇ each independently represent an uncharged polar amino acid or a hydrophobic amino acid, and U represents an uncharged polar amino acid, and
   the olfactory receptor protein satisfies at least one condition selected from the group consisting of the following conditions 1 to 3:
      condition 1: in the amino acid sequence B,
         X₃ and/or X₄ is substituted, and
         X₃ is a branched-chain amino acid and/or X₄ is an uncharged polar amino acid,
      condition 2: if X₅ and X₆ in the amino acid sequence A are both a positively charged polar amino acid, X₆ in the amino acid sequence B is an amino acid other than a positively charged polar amino acid, and
      condition 3:
         if either X₁ or X₂ or both in the amino acid sequence A are a positively charged polar amino acid,
         in the amino acid sequence B,
            X₁ and/or X₂ is substituted, and
            X₁ is an uncharged polar amino acid and/or X₂ is a hydrophobic amino acid.
Item 6. An olfactory receptor protein comprising
   (1) amino acid sequence 1C composed of amino acid sequence 1A of SEQ ID NO: 1 or amino acid sequence 1B with at least 80% identity to the amino acid sequence 1A, the amino acid sequence 1A and the amino acid sequence 1B each containing a mutation, wherein the amino acid sequence 1C contains mutation 1a, and the mutation 1a is deletion of a partial or entire region of the first extracellular loop;
   (2) amino acid sequence 2C composed of amino acid sequence 2A of SEQ ID NO: 2 or amino acid sequence 2B with at least 80% identity to the amino acid sequence 2A, the amino acid sequence 2A and the amino acid sequence 2B each containing a mutation,
      wherein the amino acid sequence 2C contains mutation 2a, and the mutation 2a is substitution 2ax or substitution 2ay,
      the substitution 2ax is substitution of at least one amino acid selected from the group consisting of L60, M138, and A152 in the amino acid sequence 2A, and
      the substitution 2ay corresponds to the substitution 2ax in the amino acid sequence 2B;
   (3) amino acid sequence 3C composed of amino acid sequence 3A of SEQ ID NO: 3 or amino acid sequence 3B with at least 80% identity to the amino acid sequence 3A, the amino acid sequence 3A and the amino acid sequence 3B each containing a mutation,
      wherein the amino acid sequence 3C contains mutation 3a and mutation 3b,
      the mutation 3a is substitution 3ax or substitution 3ay,
      the substitution 3ax is substitution of amino acids S114, A120, H162, V186, V352, and S368 in the amino acid sequence 3A, and the substitution 3ay corresponds to the substitution 3ax in the amino acid sequence 3B,
      the mutation 3b is substitution of a charged amino acid into an uncharged amino acid in a region from the third extracellular loop to the third intracellular loop; or
   (4) amino acid sequence 4C composed of amino acid sequence 4A of SEQ ID NO: 4 or amino acid sequence 4B with at least 80% identity to the amino acid sequence 4A, the amino acid sequence 4A and the amino acid sequence 4B each containing a mutation,
      wherein the amino acid sequence 4C contains mutation 4a, and the mutation 4a is substitution 4ax or substitution 4ay,
      the substitution 4ax is substitution of amino acids M61, C104, R112, G181, V189, and I327 in the amino acid sequence 4A, and the substitution 4ay corresponds to the substitution 4ax in the amino acid sequence 4B.
Item 7. A polynucleotide comprising a coding sequence of the olfactory receptor protein of Item 6.
Item 8. A cell comprising the polynucleotide of Item 7.
Item 9. A non-human animal comprising the cell according to Item 8.
Item 10. A nitro compound detection sensor comprising a lipid bilayer, a cell, or a non-human animal containing the cell, the lipid bilayer and the cell each containing the nitro compound detection element of any one of Items 1 to 5.
Item 10A. Use of the lipid bilayer, the cell, or the non-human animal containing the cell in a nitro compound detection sensor, the lipid bilayer and the cell each containing the nitro compound detection element of any one of Items 1 to 5.
Item 10B. Use of the lipid bilayer, the cell, or the non-human animal containing the cell in the production of a nitro compound detection sensor, the lipid bilayer and the cell each containing the nitro compound detection element of any one of Items 1 to 5.
Item 11. A method for detecting a nitro compound, comprising brining the nitro compound detection element of any one of Items 1 to 5 or the nitro compound detection sensor of Item 10 into contact with a nitro compound.

### Advantageous Effects of Invention

The present disclosure provides a technique for detecting a nitro compound.

### Brief Description of Drawings

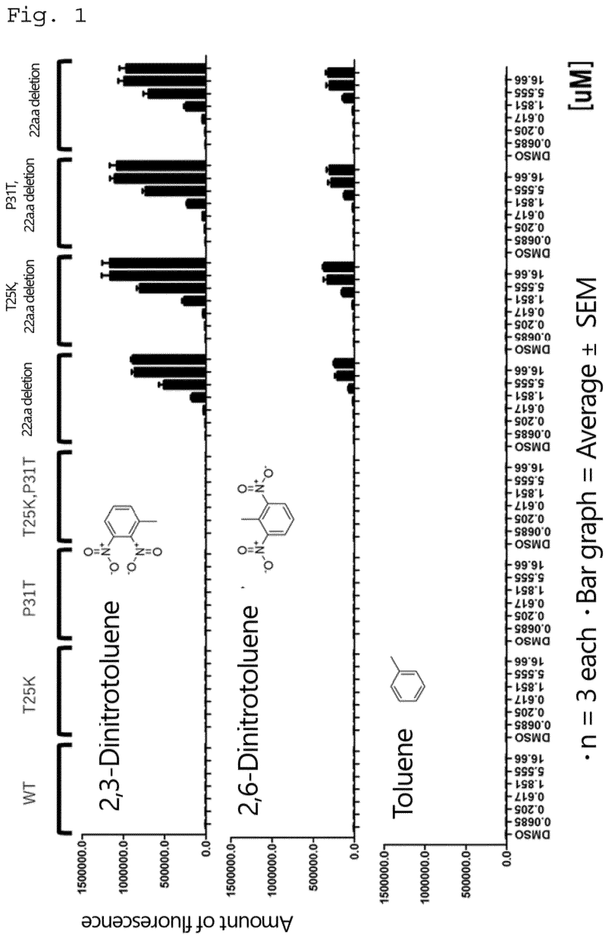

Fig. 1 shows the results of measuring the response activity of *Anopheles* olfactory receptor 28 and mutants thereof to nitro compounds (2,3-dinitrotoluene and 2,6-dinitrotoluene) (Test Example 1). The ordinate shows the amount of fluorescence (= activity intensity), whereas the abscissa shows the concentration of each test substance in culture medium. The olfactory receptors used are shown in the upper part of the graph (wherein WT indicates the wild type and the others indicate mutation sites).

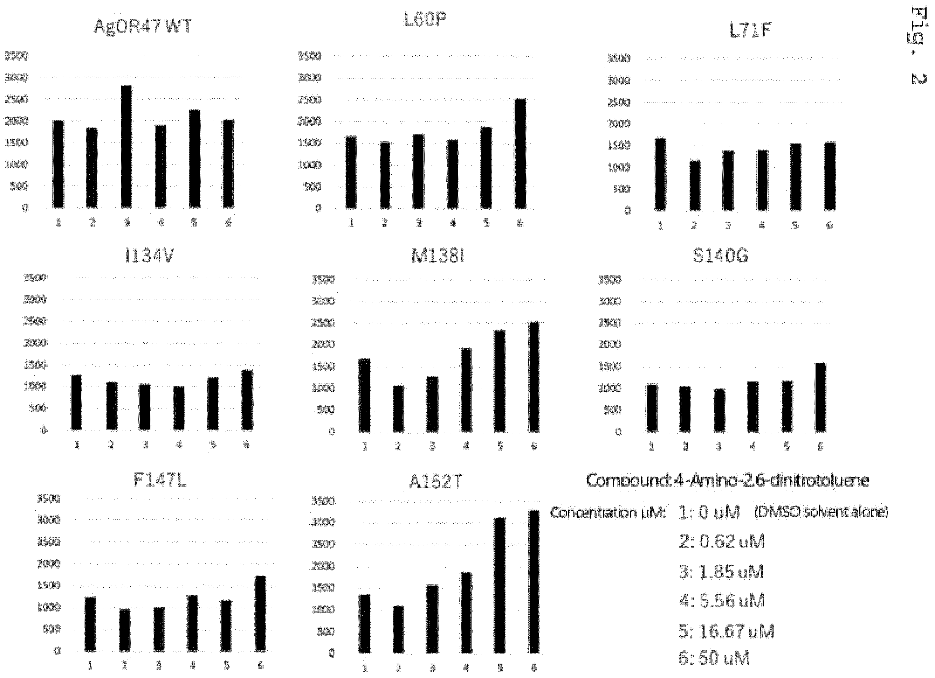

Fig. 2 shows the result of measuring the response activity of *Anopheles* olfactory receptor 47 and mutants thereof to a nitro compound (4-amino-2,6-dinitrotoluene) (Test Example 2). The ordinate shows the amount of fluorescence (= activity intensity), whereas the abscissa shows the concentration of the test substance in culture medium (see the graph legend). The olfactory receptors used are shown at the top of the graph (wherein WT indicates wild type and the others indicate mutation sites).

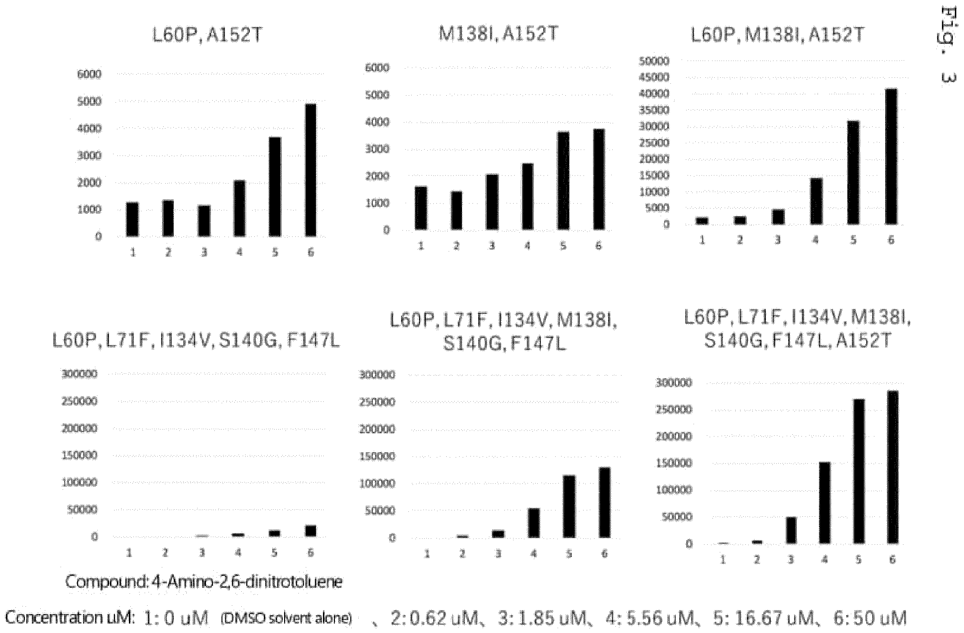

Fig. 3 shows the result of measuring the response activity of *Anopheles* olfactory receptor 47 and mutants thereof to a nitro compound (4-amino-2,6-dinitrotoluene) (Test Example 2). The ordinate shows the amount of fluorescence (= activity intensity), whereas the abscissa shows the concentration of the test substance in the culture medium (see the graph legend). The mutation sites of the olfactory receptors used are shown at the top of the graph.

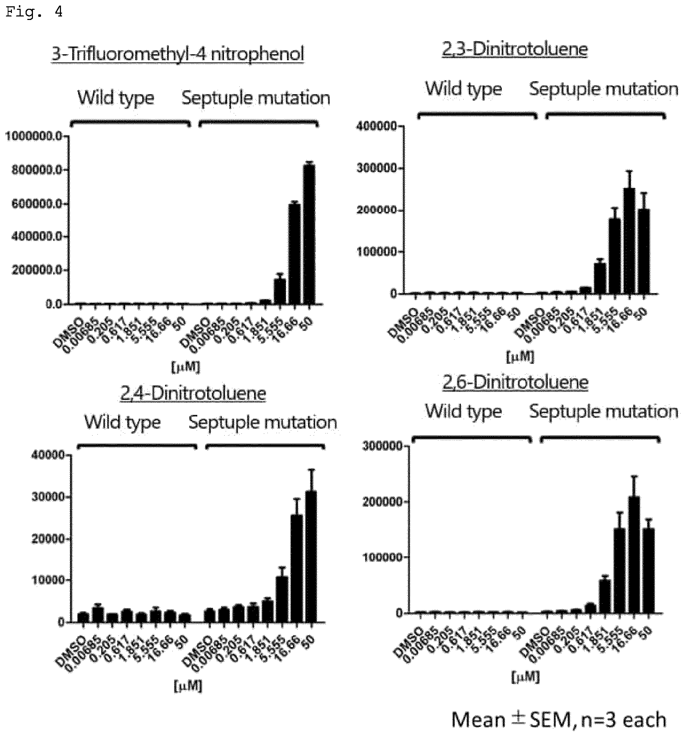

Fig. 4 shows the result of measuring the response activity of *Anopheles* olfactory receptor 47 and a mutant thereof to nitro compounds (3-trifluoromethyl-4-nitrophenol, 2,3-dinitrotoluene, 2,4-dinitrotoluene, or 2,6-dinitrotoluene) (Test Example 2). The ordinate shows the amount of fluorescence (= activity intensity), whereas the abscissa shows the concentration of each test substance in culture medium. The olfactory receptors used (wild type or septuple mutant (Example 12)) are shown at the top of the graph.

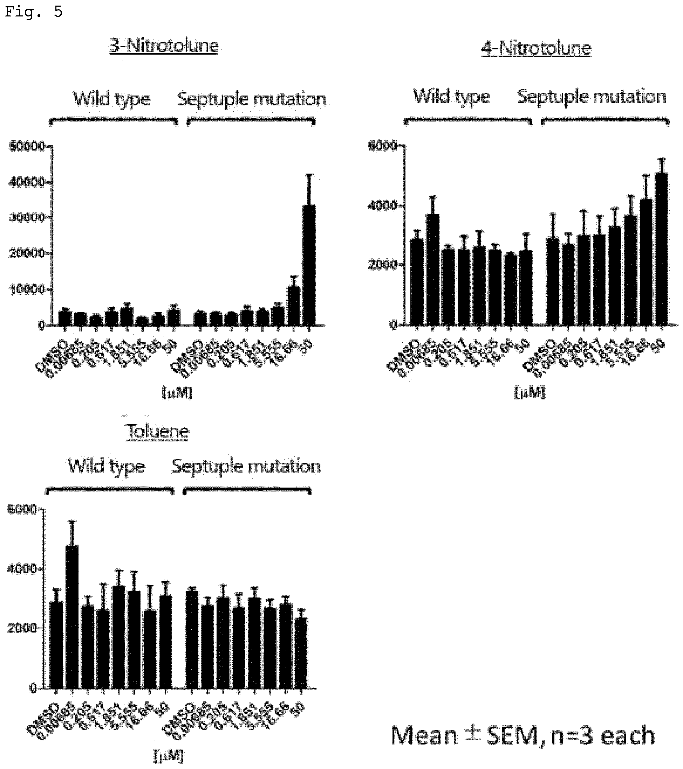

Fig. 5 shows the result of measuring the response activity of *Anopheles* olfactory receptor 47 and a mutant thereof to nitro compounds (3-nitrotoluene, 4-nitrotoluene, or toluene) (Test Example 2). The ordinate shows the amount of fluorescence (= activity intensity), whereas the abscissa shows the concentration of the test substance in culture medium. The olfactory receptors used (wild type or septuple mutant (Example 12)) are shown at the top of the graph.

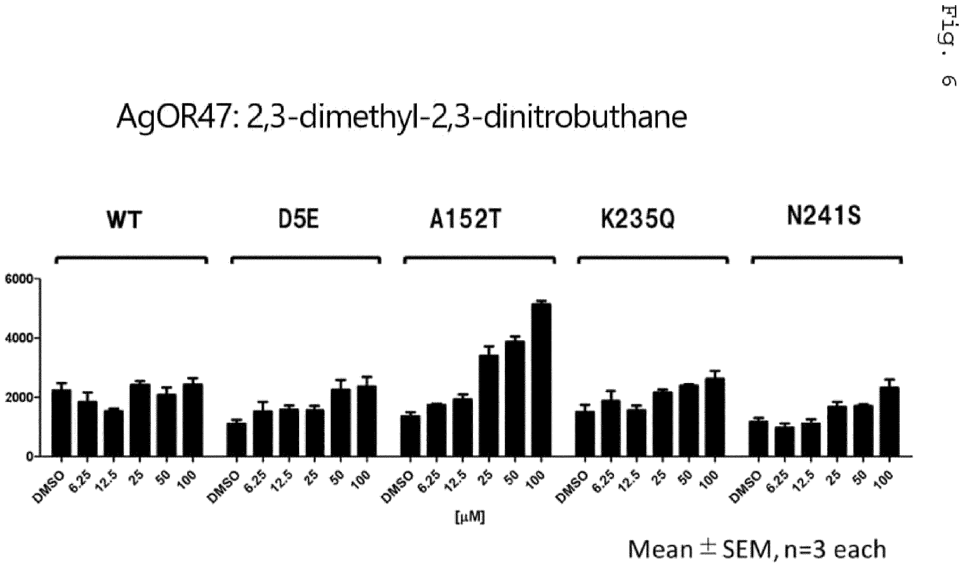

Fig. 6 shows the result of measuring the response activity of *Anopheles* olfactory receptor 47 and mutants thereof to a nitro compound (2,3-dimethyl-2,3-dinitrobutane) (Test Example 2). The ordinate shows the amount of fluorescence (= activity intensity), whereas the abscissa shows the concentration of the test substance in culture medium (DMSO indicates 0 µM). The olfactory receptors used are shown at the top of the graph (wherein WT indicates the wild type, and the others indicate mutation sites).

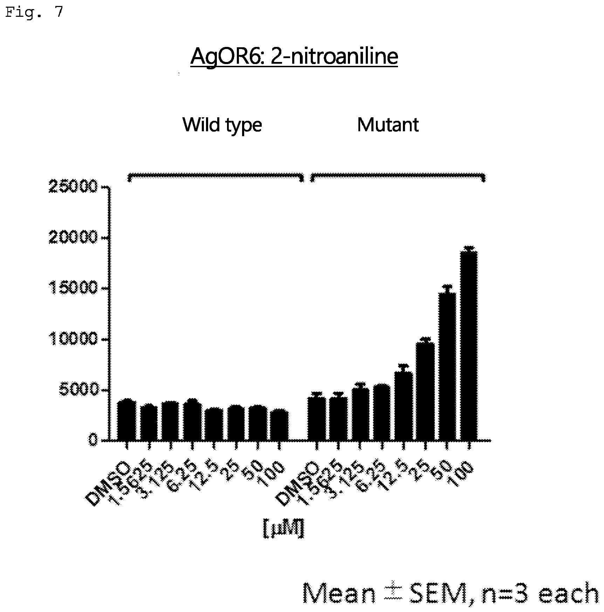

Fig. 7 shows the result of measuring the response activity of *Anopheles* olfactory receptor 6 and a mutant thereof to a nitro compound (2-nitroaniline) (Test Example 3). The ordinate shows the amount of fluorescence (= activity intensity), whereas the abscissa shows the concentration of the test substance in culture medium (DMSO indicates 0 µM). The olfactory receptors used (the wild type and mutant) are shown at the top of the graph.

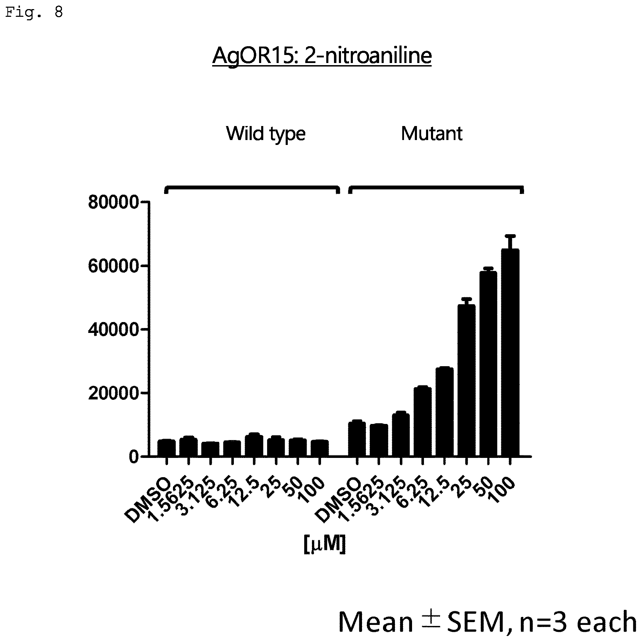

Fig. 8 shows the result of measuring the response activity of *Anopheles* olfactory receptor 15 and a mutant thereof to a nitro compound (2-nitroaniline) (Test Example 4). The ordinate shows the amount of fluorescence (= activity intensity), whereas the abscissa shows the concentration of the test substance in culture medium (DMSO indicates 0 µM). The olfactory receptors used (wild type and mutants) are shown at the top of the graph.

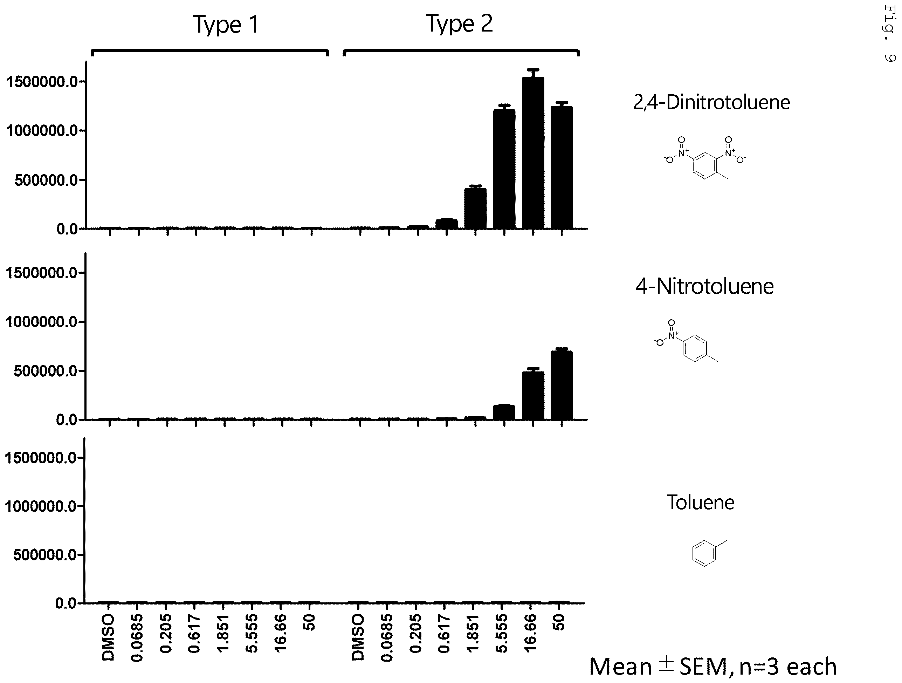

Fig. 9 shows the result of measuring the response activity of *Aedes* olfactory receptor 4 and a mutants thereof to nitro compounds (2,4-dinitrotoluene and 4-nitrotoluene) (Test Example 5). The ordinate shows the amount of fluorescence (= activity intensity), whereas the abscissa shows the concentration of the test substance in culture medium. The olfactory receptors used are shown at the top of the graph.

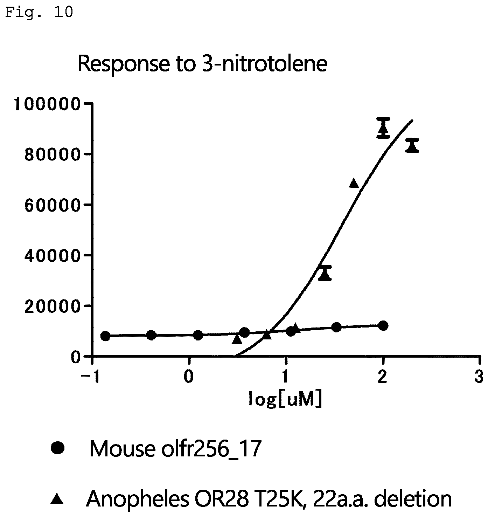

Fig. 10 shows the result of measuring the response activity of *Anopheles* olfactory receptor 28 and mouse olfactory receptor Olfr256_17 to a nitro compound (3-nitrotoluene) (Test Example 6). The ordinate shows the amount of fluorescence (= activity intensity), whereas the abscissa shows the concentration of the test substance in culture medium.

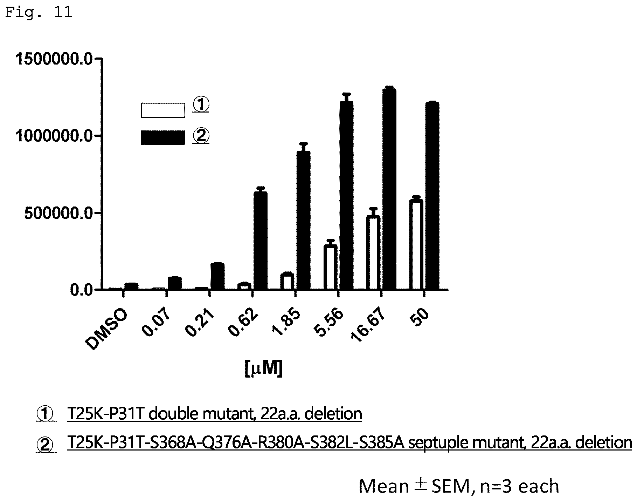

Fig. 11 shows the results of measuring the response activity of *Anopheles* olfactory receptor 28 further containing an amino acid substitution in the seven-transmembrane domain to a nitro compound (2,3-dinitrotoluene) (Test Example 7). The ordinate shows the amount of fluorescence (= activity intensity), whereas the abscissa shows the concentration of the test substance in culture medium (DMSO indicates 0 µM).

### Description of Embodiments

### 1. Definition

In the present specification, the terms "comprising," "containing," and "including" include the concepts of comprising, containing, consisting essentially of, and consisting of.

The "identity" of amino acid sequences refers to the degree to which two or more contrastable amino acid sequences match each other. Thus, the higher the degree of match between two amino acid sequences, the higher the identity or similarity of those sequences. The level of amino acid sequence identity is determined, for example, by using FASTA, which is a tool for sequence analysis, with default parameters. Alternatively, the level of amino acid sequence identity can be determined by using the BLAST algorithm by Karlin and Altschul (Karlin S, Altschul SF. Methods for assessing the statistical significance of molecular sequence features by using general scorings schemes, Proc Natl Acad Sci USA. 87: 2264-2268 (1990), Karlin S, Altschul SF. Applications and statistics for multiple high-scoring segments in molecular sequences, Proc Natl Acad Sci USA. 90: 5873-7 (1993)). A program called "BLASTX," based on this BLAST algorithm, has been developed. The specific techniques of these analysis methods are known and can be found on the website of the National Center of Biotechnology Information (NCBI) (http://www.ncbi.nlm.nih.gov/). The "identity" of base sequences is also defined in the same manner as above.

In the present specification, "conservative substitution" means the substitution of an amino acid residue with an amino acid residue having a similar side chain. For example, the substitution between amino acid residues having a basic side chain such as lysine, arginine, or histidine is considered to be a conservative substitution. The following substitutions between other amino acid residues are also considered to be a conservative substitution: the substitution between amino acid residues having an acidic side chain such as aspartic acid or glutamic acid; the substitution between amino acid residues having an uncharged polar side chain such as glycine, asparagine, glutamine, serine, threonine, tyrosine, or cysteine; the substitution between amino acid residues having a nonpolar side chain such as alanine, valine, leucine, isoleucine, proline, phenylalanine, methionine, or tryptophan; the substitution between amino acid residues having a β-branched side chain such as threonine, valine, or isoleucine; and the substitution between amino acid residues having an aromatic side chain such as tyrosine, phenylalanine, tryptophan, or histidine.

In the present specification, nucleotides such as DNA and RNA may have known chemical modification as illustrated below. To prevent the degradation by hydrolases such as nucleases, the phosphate residue (phosphate) of each nucleotide can be substituted with a chemically modified phosphate residue, such as phosphorothioate (PS), methylphosphonate, or phosphorodithionate. The hydroxyl group at position 2 of the ribose of each ribonucleotide may also be substituted with -OR (R indicates, for example, CH₃(2'-O-Me), CH₂CH₂OCH₃(2'-O-MOE), CH₂CH₂NHC(NH)NH₂, CH₂CONHCH₃, or CH₂CH₂CN). Additionally, the base moiety (pyrimidine, purine) may be chemically modified, by, for example, introduction of a methyl group or a cationic functional group into position 5 of the pyrimidine base, or substitution of the carbonyl group at position 2 with thiocarbonyl. Additionally, the polynucleotide of the present invention also includes, but is not limited to, those formed by modifying the phosphate moiety or the hydroxyl portion, for example, by biotin, an amino group, a lower alkyl amine group, or an acetyl group. The polynucleotide for use can also be, for example, preferably BNA (LNA), which is prepared by crosslinking the 2' oxygen and the 4' carbon in the ribose moiety of a nucleotide to fix the ribose moiety in N-conformation.

The olfactory receptor protein is a membrane protein with a seven-transmembrane structure, which is sequentially organized from the following elements linked in order from the amino terminus ("N-terminus" below) to the carboxyl terminus ("C-terminus" below): the N-terminal domain (NT), the first transmembrane domain (TM1), the first extracellular loop (EC1), the second transmembrane domain (TM2), the first intracellular loop (IC1), the third transmembrane domain (TM3), the second extracellular loop (EC2), the fourth transmembrane domain (TM4), the second intracellular loop (IC2), the fifth transmembrane domain (TM5), the third extracellular loop (EC3), the sixth transmembrane domain (TM6), the third intracellular loop (IC3), the seventh transmembrane domain (TM7), and the C-terminal domain (CT). In the present disclosure, each domain is determined by structure prediction using TMPred with conditions set to default (K. Hofmann, W. Stoffel, TMbase - a database of membrane spanning proteins segments, Biol. Chem. Hoppe-Seyler, 374 (1993), p. 166, https://embnet.vital-it.ch/software/TMPRED_form.html).

In the present specification, the mutation of an amino acid is specifically deletion, substitution, insertion, or addition of an amino acid. The olfactory receptor protein may be deficient with an amino acid that has little effect on chemical substance response activity, such as an olfactory receptor peptide deficient with some amino acids other than the transmembrane domain.

In the present specification, the position of an amino acid in an amino acid sequence may be indicated by the alphabet indicating an amino acid position + the amino acid number from the amino acid at the N-terminus. For example, "A153" indicates alanine at the 153rd position from the N-terminus.

In the present specification, the phrase "nitro compound responsiveness" refers to the properties that an olfactory receptor recognizes a nitro compound, and then an olfactory receptor complex formed from the olfactory receptor and an olfactory receptor co-receptor is activated to show ion channel activity. The nitro compound responsiveness of an olfactory receptor can be measured with the ion channel activity of an olfactory receptor complex formed from the olfactory receptor that has come into contact with a nitro compound and an olfactory receptor co-receptor as an index. For example, cells expressing (a) an olfactory receptor, (b) an olfactory receptor co-receptor, or (c) a protein that develops color or emits light due to ions (e.g., calcium ions) flown into the cells upon response of an olfactory receptor are brought into contact with a nitro compound, and the amount of luminescence of the cells is measured. As the amount of luminescence measured is higher, the nitro compound responsiveness of the olfactory receptor to the chemical substance is determined to be higher. Specific examples of the measurement of nitro compound responsiveness are disclosed in Test Example 1-2 and Test Example 1-3, described later.

### 2. Olfactory Receptor Protein

In an embodiment, the present disclosure relates to an olfactory receptor protein comprising
(1) amino acid sequence 1C composed of amino acid sequence 1A of SEQ ID NO: 1 or amino acid sequence 1B with at least 80% identity to the amino acid sequence 1A, the amino acid sequence 1A and the amino acid sequence 1B each containing a mutation, wherein the amino acid sequence 1C contains mutation 1a, and the mutation 1a is deletion of a partial or entire region of the first extracellular loop;
(2) amino acid sequence 2C composed of amino acid sequence 2A of SEQ ID NO: 2 or amino acid sequence 2B with at least 80% identity to the amino acid sequence 2A, the amino acid sequence 2A and the amino acid sequence 2B each containing a mutation,
   wherein the amino acid sequence 2C contains mutation 2a, and the mutation 2a is substitution 2ax or substitution 2ay,
   the substitution 2ax is substitution of at least one amino acid selected from the group consisting of L60, M138, and A152 in the amino acid sequence 2A, and
   the substitution 2ay corresponds to the substitution 2ax in the amino acid sequence 2B;
(3) amino acid sequence 3C composed of amino acid sequence 3A of SEQ ID NO: 3 or amino acid sequence 3B with at least 80% identity to the amino acid sequence 3A, the amino acid sequence 3A and the amino acid sequence 3B each containing a mutation,
   wherein the amino acid sequence 3C contains mutation 3a and mutation 3b,
   the mutation 3a is substitution 3ax or substitution 3ay,
   the substitution 3ax is substitution of amino acids S114, A120, H162, V186, V352, and S368 in the amino acid sequence 3A, and the substitution 3ay corresponds to the substitution 3ax in the amino acid sequence 3B,
   the mutation 3b is substitution of a charged amino acid into an uncharged amino acid in a region from the third extracellular loop to the third intracellular loop;
(4) amino acid sequence 4C composed of amino acid sequence 4A of SEQ ID NO: 4 or amino acid sequence 4B with at least 80% identity to the amino acid sequence 4A, the amino acid sequence 4A and the amino acid sequence 4B each containing a mutation,
   wherein the amino acid sequence 4C contains mutation 4a, and the mutation 4a is substitution 4ax or substitution 4ay,
   the substitution 4ax is substitution of amino acids M61, C104, R112, G181, V189, and I327 in the amino acid sequence 4A, and the substitution 4ay corresponds to the substitution 4ax in the amino acid sequence 4B; or
(5) amino acid sequence 5C composed of amino acid sequence 5A of SEQ ID NO: 5 or amino acid sequence 5B with at least 80% identity to the amino acid sequence 5A, the amino acid sequence 5A and the amino acid sequence 5B each containing a mutation,
   wherein the amino acid sequence 5C contains mutation 5a, and the mutation 5a is substitution 5ax or substitution 5ay,
   the substitution 5ax is substitution of amino acid L178 in the amino acid sequence 5A, and the substitution 5ay corresponds to the substitution 5ax in the amino acid sequence 5B (which may be referred to as "the olfactory receptor protein of the present disclosure" in the present specification). The following describes the olfactory receptor protein.

### 2-1. Amino Acid Sequence 1C

Amino acid sequence 1C can be any amino acid sequence that is composed of amino acid sequence 1A of SEQ ID NO: 1 or amino acid sequence 1B with at least 80% identity to the amino acid sequence 1A, the amino acid sequence 1A and amino acid sequence 1B each containing a mutation, and that contains mutation 1a, which is deletion of a partial or entire region of the first extracellular loop.

The amino acid sequence 1A is the amino acid sequence of olfactory receptor 28 that is intrinsically expressed (inherently possessed) by *Anopheles gambiae.*

The amino acid sequence 1B can be any amino acid sequence as long as the nitro compound responsiveness of a protein composed of a sequence containing the amino acid sequence 1B having the mutation 1a introduced (protein 1B) is not significantly reduced compared with the nitro compound responsiveness of a protein composed of a sequence containing the amino acid sequence 1A having the mutation 1a introduced (protein 1A). The nitro compound responsiveness of the protein 1B is, for example, 50% or more, preferably 60% or more, more preferably 70% or more, still more preferably 80% or more, and yet more preferably 90% or more, of the nitro compound responsiveness of the protein 1A taken as 100%. From this viewpoint, the identity of the amino acid sequence 1B to the amino acid sequence 1A is preferably at least 85%, more preferably at least 90%, still more preferably at least 95%, yet more preferably at least 97%, particularly preferably at least 99%, and less than 100%.

The amino acid sequence 1B is preferably the amino acid sequence described in (1B'): (1B') Amino acid sequence 1B' containing the amino acid sequence 1A with one or multiple amino acid mutations.

In the amino acid sequence 1B', "multiple" means, for example, 2 to 20, preferably 2 to 10, more preferably 2 to 5, still more preferably 2 to 3, and yet more preferably 2.

The amino acid mutation introduced into the amino acid sequence 1A contained in the amino acid sequence 1B is, for example, substitution, deletion, addition, or insertion, preferably substitution, and particularly more preferably conservative substitution.

The amino acid sequence 1B is preferably the amino acid sequence of an olfactory receptor derived from an insect. The term "derived" indicates that the sequence is one intrinsically expressed (inherently possessed) by the organism. The insect is preferably an insect in the superorder *Endopterygota,* more preferably an insect in the order *Diptera* such as the family *Culicidae* and the family *Drosophilidae,* an insect in the order *Lepidoptera* such as the family *Bombycidae,* and an insect in the order *Hymenoptera* such as the family *Apidae;* more preferably an insect in the order *Dipterainsect,* such as the family *Culicidae,* and the family *Drosophilidae;* and still more preferably an insect in the family *Culicidae.* Examples of insects in the family *Culicidae* include *Anopheles gambiae, Aedes aegypti,* and *Culex quinquefasciatus.* Examples of insects in the family *Drosophilidae* include *Drosophila melanogaster, Drosophila pseudoobscura,* and *Drosophila virillis.* Examples of insects in the family *Bombycidae* include *Bombyx mori, Bombyx mandarina,* and *Trilocha varians.* Examples of insects in the family *Apidae* include *Apis mellifera, Apis florea, Apis dorsata,* and *Bombus terrestris.*

The mutation 1a is deletion of a partial region or the entire region of the first extracellular loop. The first extracellular loop is the region of 30 amino acids from the 55th amino acid to the 84th amino acid from the N-terminus in the amino acid sequence 1A. The mutation 1a is more preferably in the region of 5 to 30 amino acids from the amino acid at the N-terminus of the first extracellular loop, more preferably the region of 10 to 25 amino acids from the amino acid at the N-terminus, and still more preferably the region of 15 to 25 amino acids from the amino acid at the N-terminus.

In the mutation 1a, it is preferred that a portion closer to the C-terminus of the first transmembrane domain, which is the region adjacent to the N-terminus of the first extracellular loop, is further deleted. For example, it is preferred that the region of 1 to 5 amino acids including the amino acid at the C-terminus of the first transmembrane domain is deleted.

The amino acid sequence 1C preferably further contains mutation 1b. The mutation 1b is substitution 1bx that is the substitution of at least one amino acid selected from the group consisting of T25 and P31 in the amino acid sequence 1A or substitution 1by that corresponds to the substitution 1bx in the amino acid sequence 1B.

The phrase "substitution ... that corresponds to ..." indicates that when two sequences are compared with BLAST (set to default), a substitution is present at the same position on each of the aligned sequences. For example, the substitution that corresponds to T25 of the amino acid sequence 1A indicates a substitution of an amino acid in the amino acid sequence 1B at the same position as that of T25 in the amino acid sequence 1A in the aligned sequences obtained in comparison between the amino acid sequence 1A and the amino acid sequence 1B. Additionally, the amino acid after substitution in the amino acid sequence 1A is identical to the amino acid after substitution in the amino acid sequence 1B.

The amino acid after mutation of T25 or the amino acid after mutation of the amino acid that corresponds to T25 in the mutation 1b is preferably a basic amino acid. Examples of basic amino acids include lysine, arginine, and histidine, with lysine being particularly preferable.

The amino acid after mutation of P31 or the amino acid after mutation of the amino acid that corresponds to P31 in the mutation 1b is preferably a hydrophilic neutral amino acid. Examples of hydrophilic neutral amino acids include serine, threonine, asparagine, and glutamine, with threonine being particularly preferable.

The amino acid sequence 1C may contain other amino acid mutations (e.g., substitution, or conservative substitution) as long as the nitro compound responsiveness is not significantly impaired. In an embodiment, the nitro compound responsiveness can be further enhanced by introducing a specific mutation into the seven-transmembrane domain. The number of other amino acid mutations is, for example, 1 to 50, 1 to 20, 1 to 10, or 1 to 5.

The amino acid sequence 1C is, specifically, for example, the amino acid sequence of SEQ ID NO: 21, 25, 27, or 29, or an amino acid sequence with at least 80% identity (preferably at least 85%, more preferably at least 90%, still more preferably at least 95%, yet more preferably at least 97%, particularly preferably at least 99%, and less than 100% identity) to the amino acid sequence.

### 2-2. Amino Acid Sequence 2C

Amino acid sequence 2C can be any amino acid sequence that is composed of amino acid sequence 2A of SEQ ID NO: 2 or amino acid sequence 2B with at least 80% identity to the amino acid sequence 2A, the amino acid sequence 2A and the amino acid sequence 2B each containing a mutation, and that contains mutation 2a, wherein the mutation 2a is substitution 2ax that is substitution of at least one amino acid selected from the group consisting of L60, M138, and A152 in the amino acid sequence 2A or substitution 2ay that corresponds to the substitution 2ax in the amino acid sequence 2B.

The amino acid sequence 2A is the amino acid sequence of olfactory receptor 47 that is intrinsically expressed (inherently possessed) by *Anopheles gambiae.*

The amino acid sequence 2B can be any amino acid sequence as long as the nitro compound responsiveness of a protein composed of a sequence containing the amino acid sequence 2B having the mutation 2a introduced (protein 2B) is not significantly reduced compared with the nitro compound responsiveness of a protein composed of a sequence containing the amino acid sequence 2A having the mutation 2a introduced (protein 2A). The nitro compound responsiveness of the protein 2B is, for example, 50% or more, preferably 60% or more, more preferably 70% or more, still more preferably 80% or more, and yet more preferably 90% or more, of the nitro compound responsiveness of the protein 2A taken as 100%. From this viewpoint, the identity of the amino acid sequence 2B to the amino acid sequence 2A is preferably at least 85%, more preferably at least 90%, still more preferably at least 95%, yet more preferably at least 97%, particularly preferably at least 99%, and less than 100%.

The amino acid sequence 2B is preferably the amino acid sequence described in (2B'): (2B') Amino acid sequence 2B' containing the amino acid sequence 2A with one or multiple amino acid mutations.

In the amino acid sequence 2B', "multiple" means, for example, 2 to 20, preferably 2 to 10, more preferably 2 to 5, still more preferably 2 to 3, and yet more preferably 2.

The amino acid mutation introduced into the amino acid sequence 2A contained in the amino acid sequence 2B is, for example, substitution, deletion, addition, or insertion, preferably substitution, and particularly more preferably conservative substitution.

The amino acid sequence 2B is preferably the amino acid sequence of an olfactory receptor derived from an insect. The olfactory receptor derived from an insect is as defined in the "2-1. Amino Acid Sequence 1C" section above.

The mutation 2a is substitution 2ax that is substitution of at least one amino acid selected from the group consisting of L60, M138, and A152 in the amino acid sequence 2A or substitution 2ay that corresponds to the substitution 2ax in the amino acid sequence 2B. The phrase "substitution ... that corresponds to ..." is as defined in the "2-1. Amino Acid Sequence 1C" section above.

The amino acid after mutation of L60 or the amino acid after mutation of the amino acid that corresponds to L60 in the mutation 2a is preferably proline.

The amino acid after mutation of M138 or the amino acid after mutation of the amino acid that corresponds to M138 in the mutation 2a is preferably a branched-chain amino acid. Examples of branched-chain amino acids include valine, isoleucine, and leucine, with isoleucine being particularly preferable.

The amino acid after mutation of A152 or the amino acid after mutation of the amino acid that corresponds to A152 in the mutation 2a is preferably a hydrophilic neutral amino acid. Examples of hydrophilic neutral amino acids include serine, threonine, asparagine, and glutamine, with threonine being particularly preferable.

The mutation 2a preferably contains substitution of at least one of M138 and A152 or substitution of the amino acid that corresponds to at least one of M138 and A152, and more preferably substitution of both M138 and A152 or substitution of both the amino acids that correspond to M138 and A152.

The amino acid sequence 2C preferably further contains mutation 2b. The mutation 2b is substitution 2bx that is substitution of at least one amino acid selected from the group consisting of D5, L71, I134, S140, F147, K235, and N241 in the amino acid sequence 2A or substitution 2by that corresponds to the substitution 2bx in the amino acid sequence 2B.

The amino acid after mutation of D5 or the amino acid after mutation of the amino acid that corresponds to D5 in the mutation 2b is preferably an acidic amino acid. Examples of acidic amino acids include aspartic acid and glutamic acid, with glutamic acid being particularly preferable.

The amino acid after mutation of L71 or the amino acid after mutation of the amino acid that corresponds to L71 in the mutation 2b is preferably an aromatic amino acid. Examples of aromatic amino acids include phenylalanine, tyrosine, and tryptophan, with phenylalanine being particularly preferable.

The amino acid after mutation of I134 or the amino acid after mutation of the amino acid that corresponds to I134 in the mutation 2b is preferably a branched-chain amino acid. Examples of branched-chain amino acids include valine, isoleucine, and leucine, with valine being particularly preferable.

The amino acid after mutation of S140 or the amino acid after mutation of the amino acid that corresponds to S140 in the mutation 2b is preferably an aliphatic amino acid. Examples of aliphatic amino acids include alanine, glycine, valine, isoleucine, and leucine, with glycine being particularly preferable.

The amino acid after mutation of F147 or the amino acid after mutation of the amino acid that corresponds to F147 in the mutation 2b is preferably a branched-chain amino acid. Examples of branched-chain amino acids include valine, isoleucine, and leucine, with leucine being particularly preferable.

The amino acid after mutation of K235 or the amino acid after mutation of the amino acid that corresponds to K235 in the mutation 2b is preferably a hydrophilic neutral amino acid. Examples of hydrophilic neutral amino acids include serine, threonine, asparagine, and glutamine, with glutamine being particularly preferable.

The amino acid after mutation of N241 or the amino acid after mutation of the amino acid that corresponds to N241 in the mutation 2b is preferably a hydrophilic neutral amino acid. Examples of hydrophilic neutral amino acids include serine, threonine, asparagine, and glutamine, with serine being particularly preferable.

The mutation 2b preferably contains substitution of L71F or substitution of the amino acid that corresponds to L71F, more preferably substitution of L71F and I134 or substitution of the amino acids that correspond to L71F and I134, still more preferably substitution of L71F, I134, and S140 or substitution of the amino acids that correspond to L71F, I134, and S140, and yet more preferably substitution of L71F, I134, S140, and F147 or substitution of the amino acids that correspond to L71F, I134, S140, and F147.

The amino acid sequence 2C may contain other amino acid mutations (e.g., substitution or conservative substitution) as long as the nitro compound responsiveness is not significantly impaired. In an embodiment, the nitro compound responsiveness can be further enhanced by introducing a specific mutation into the seven-transmembrane domain. The number of other amino acid mutations is, for example, 1 to 50, 1 to 20, 1 to 10, or 1 to 5.

The amino acid sequence 2C is, specifically, for example, the amino acid sequence of SEQ ID NO: 63, 67, 71, 73, 75, 77, or 79, or an amino acid sequence with at least 80% identity (preferably, at least 85%, more preferably at least 90%, still more preferably at least 95%, yet more preferably at least 97%, particularly preferably at least 99%, and less than 100% identity) to the amino acid sequence.

### 2-3. Amino Acid Sequence 3C

Amino acid sequence 3C can be any amino acid sequence that is composed of amino acid sequence 3A of SEQ ID NO: 3 or amino acid sequence 3B with at least 80% identity to the amino acid sequence 3A, the amino acid sequence 3A and the amino acid sequence 3B each containing a mutation, and that contains mutation 3a and mutation 3b, wherein the mutation 3a is substitution 3ax or substitution 3ay, the substitution 3ax is substitution of amino acids S114, A120, H162, V186, V352, and S368 in the amino acid sequence 3A, the substitution 3ay corresponds to the substitution 3ax in the amino acid sequence 3B, and the mutation 3b is substitution of a charged amino acid into an uncharged amino acid in a region from the third extracellular loop to the third intracellular loop.

The amino acid sequence 3A is the amino acid sequence of olfactory receptor 6 that is intrinsically expressed (inherently possessed) by *Anopheles gambiae.*

The amino acid sequence 3B can be any amino acid sequence as long as the nitro compound responsiveness of a protein composed of a sequence containing the amino acid sequence 3B having the mutation 3a and mutation 3b introduced (protein 3B) is not significantly reduced compared with the nitro compound responsiveness of a protein composed of a sequence containing the amino acid sequence 3A having the mutation 3a and mutation 3b introduced (protein 3A). The nitro compound responsiveness of the protein 3B is, for example, 50% or more, preferably 60% or more, more preferably 70% or more, still more preferably 80% or more, and yet more preferably 90% or more, of the nitro compound responsiveness of the protein 3A taken as 100%. From this viewpoint, the identity of the amino acid sequence 3B to the amino acid sequence 3A is preferably at least 85%, more preferably at least 90%, still more preferably at least 95%, yet more preferably at least 97%, particularly preferably at least 99%, and less than 100%.

The amino acid sequence 3B is preferably the amino acid sequence described in (3B'): (3B') Amino acid sequence 3B' containing the amino acid sequence 3A with one or multiple amino acid mutations.

In the amino acid sequence 3B', "multiple" means, for example, 2 to 20, preferably 2 to 10, more preferably 2 to 5, still more preferably 2 to 3, and yet more preferably 2.

The amino acid mutation introduced into the amino acid sequence 3A contained in the amino acid sequence 3B is, for example, substitution, deletion, addition, or insertion, preferably substitution, and particularly more preferably conservative substitution.

The amino acid sequence 3B is preferably the amino acid sequence of an olfactory receptor derived from an insect. The olfactory receptor derived from an insect is as defined in the "2-1. Amino Acid Sequence 1C" section above.

The mutation 3a is substitution 3ax that is substitution of amino acids S114, A120, H162, V186, V352, and S368 in the amino acid sequence 3A or substitution 3ay that corresponds to the substitution 3ax in the amino acid sequence 3B. The phrase "substitution ... that corresponds to ..." is as defined in the "2-1. Amino Acid Sequence 1C" section above.

The amino acid after mutation of S114 or the amino acid after mutation of the amino acid that corresponds to S114 in the mutation 3a is preferably an aliphatic amino acid. Examples of aliphatic amino acids include alanine, glycine, valine, isoleucine, and leucine, with alanine being preferable.

The amino acid after mutation of A120 or the amino acid after mutation of the amino acid that corresponds to A120 in the mutation 3a is preferably an acidic amino acid. Examples of acidic amino acids include aspartic acid and glutamic acid, with glutamic acid being particularly preferable.

The amino acid after mutation of H162 or the amino acid after mutation of the amino acid that corresponds to H162 in the mutation 3a is preferably an aromatic amino acid. Examples of aromatic amino acids include phenylalanine, tyrosine, and tryptophan, with tyrosine being particularly preferable.

The amino acid after mutation of V186 or the amino acid after mutation of the amino acid that corresponds to V186 in the mutation 3a is preferably an aliphatic amino acid. Examples of aliphatic amino acids include alanine, glycine, valine, isoleucine, and leucine, with alanine being particularly preferable.

The amino acid after mutation of V352 or the amino acid after mutation of the amino acid that corresponds to V352 in the mutation 3a is preferably a sulfur-containing amino acid. Examples of sulfur-containing amino acids include methionine and cysteine, with methionine being particularly preferable.

The amino acid after mutation of S368 or the amino acid after mutation of the amino acid that corresponds to S368 in the mutation 3a is preferably proline.

The mutation 3b is substitution of a charged amino acid into an uncharged amino acid in a region from the third extracellular loop to the third intracellular loop (i.e., the region composed of the third extracellular loop, the sixth transmembrane domain, and the third intracellular loop). In the amino acid sequence 3A, the third extracellular loop is the region of 69 amino acids from the 211st amino acid to the 279th amino acid from the N-terminus. In the amino acid sequence 3A, the sixth transmembrane domain is the region of 23 amino acids from the 280th amino acid to the 302nd amino acid from the N-terminus. In the amino acid sequence 3A, the third intracellular loop is the region of 76 amino acids from the 303rd amino acid to the 378th amino acid from the N-terminus.

Examples of charged amino acids are acidic and basic amino acids such as lysine, arginine, histidine, aspartic acid, and glutamic acid. In the mutation 3b, the charged amino acids are partially or fully substituted with uncharged amino acids. The uncharged amino acids may be any uncharged amino acids as long as they are amino acids other than charged amino acids. The number of substitutions into uncharged amino acids is generally multiple, for example 5 to 25 or more, preferably 6 to 18, more preferably 8 to 14.

The amino acid sequence 3C may contain other amino acid mutations (e.g., substitution or conservative substitution) as long as the nitro compound responsiveness is not significantly impaired. In an embodiment, the nitro compound responsiveness can be further enhanced by introducing a specific mutation into the seven-transmembrane domain. The number of other amino acid mutations is, for example, 1 to 60, 1 to 20, 1 to 10, or 1 to 5.

The amino acid sequence 3C is, specifically, for example, the amino acid sequence of SEQ ID NO: 81 or an amino acid sequence with at least 80% identity (preferably at least 85%, more preferably at least 90%, still more preferably at least 95%, yet more preferably at least 97%, particularly preferably at least 99%, and less than 100% identity) to the amino acid sequence.

### 2-4. Amino Acid Sequence 4C

Amino acid sequence 4C can be any amino acid sequence that is composed of amino acid sequence 4A of SEQ ID NO: 4 or amino acid sequence 4B with at least 80% identity to the amino acid sequence 4A, the amino acid sequence 4A and amino acid sequence 4B each containing a mutation, and that contains mutation 4a, wherein the mutation 4a is substitution 4ax that is substitution of amino acids M61, C104, R112, G181, V189, and I327 in the amino acid sequence 4A or substitution 4ay that corresponds to the substitution 4ax in the amino acid sequence 4B.

The amino acid sequence 4A is the amino acid sequence of olfactory receptor 15 that is intrinsically expressed (inherently possessed) by *Anopheles gambiae.*

The amino acid sequence 4B can be any amino acid sequence as long as the nitro compound responsiveness of a protein composed of a sequence containing the amino acid sequence 4B having the mutation 4a introduced (protein 4B) is not significantly reduced compared with the nitro compound responsiveness of a protein composed of a sequence containing the amino acid sequence 4A having the mutation 4a introduced (protein 4A). The nitro compound responsiveness of the protein 4B is, for example, 50% or more, preferably 60% or more, more preferably 70% or more, still more preferably 80% or more, and yet more preferably 90% or more, of the nitro compound responsiveness of the protein 4A taken as 100%. From this viewpoint, the identity of the amino acid sequence 4B to the amino acid sequence 4A is preferably at least 85%, more preferably at least 90%, still more preferably at least 95%, yet more preferably at least 97%, particularly preferably at least 99%, and less than 100%.

The amino acid sequence 4B is preferably the amino acid sequence described in (4B'): (4B') Amino acid sequence 4B' containing the amino acid sequence 4A with one or multiple amino acid mutations.

In the amino acid sequence 4B', "multiple" means, for example, 2 to 20, preferably 2 to 10, more preferably 2 to 5, still more preferably 2 to 3, and yet more preferably 2.

The amino acid mutation introduced into the amino acid sequence 4A contained in the amino acid sequence 4B is, for example, substitution, deletion, addition, or insertion, preferably substitution, and particularly more preferably conservative substitution.

The amino acid sequence 4B is preferably the amino acid sequence of an olfactory receptor derived from an insect. The olfactory receptor derived from an insect is as defined in the "2-1. Amino Acid Sequence 1C" section above.

The mutation 4a is substitution 4ax that is substitution of amino acids M61, C104, R112, G181, V189, and I327 in the amino acid sequence 4A or substitution 4ay that corresponds to the substitution 4ax in the amino acid sequence 4B. The phrase "substitution ... that corresponds to ..." is as defined in the "2-1. Amino Acid Sequence 1C" section above.

The amino acid after mutation of M61 or the amino acid after mutation of the amino acid that corresponds to M61 in the mutation 4a is preferably a hydrophilic neutral amino acid. Examples of hydrophilic neutral amino acids include serine, threonine, asparagine, and glutamine, with threonine being particularly preferable.

The amino acid after mutation of C104 or the amino acid after mutation of the amino acid that corresponds to C104 in the mutation 4a is preferably a basic amino acid. Examples of basic amino acids include lysine, arginine, and histidine, with arginine being particularly preferable.

The amino acid after mutation of R112 or the amino acid after mutation of the amino acid that corresponds to R112 in the mutation 4a is preferably a hydrophilic neutral amino acid. Examples of hydrophilic neutral amino acids include serine, threonine, asparagine, and glutamine, with glutamine being particularly preferable.

The amino acid after mutation of G181 or the amino acid after mutation of the amino acid that corresponds to G181 in the mutation 4a is preferably an aliphatic amino acid. Examples of aliphatic amino acids include alanine, glycine, valine, isoleucine, and leucine, with alanine being particularly preferable.

The amino acid after mutation of V189 or the amino acid after mutation of the amino acid that corresponds to V189 in the mutation 4a is preferably a branched-chain amino acid. Examples of branched-chain amino acids include valine, isoleucine, and leucine, with isoleucine being particularly preferable.

The amino acid after mutation of I327 or the amino acid after mutation of the amino acid that corresponds to I327 in the mutation 4a is preferably a branched-chain amino acid. Examples of branched-chain amino acids include valine, isoleucine, and leucine, with valine being particularly preferable.

The amino acid sequence 4C may contain other amino acid mutations (e.g., substitution or conservative substitution) as long as the nitro compound responsiveness is not significantly impaired. In an embodiment, the nitro compound responsiveness can be further enhanced by introducing a specific mutation into the seven-transmembrane domain. The number of other amino acid mutations is, for example, 1 to 50, 1 to 20, 1 to 10, or 1 to 5.

The amino acid sequence 4C is, specifically, for example, the amino acid sequence of SEQ ID NO: 83 or an amino acid sequence with at least 80% identity (preferably at least 85%, more preferably at least 90%, still more preferably at least 95%, yet more preferably at least 97%, particularly preferably at least 99%, and less than 100% identity) to the amino acid sequence.

### 2-5. Amino Acid Sequence 5C

Amino acid sequence 5C can be any amino acid sequence that is composed of amino acid sequence 5A of SEQ ID NO: 5 or amino acid sequence 5B with at least 80% identity to the amino acid sequence 5A, the amino acid sequence 5A and the amino acid sequence 5B each containing a mutation, and that contains mutation 5a, wherein the mutation 5a is substitution 5ax that is substitution of amino acid L178 in the amino acid sequence 5A or substitution 5ay that corresponds to the substitution 5ax in the amino acid sequence 5B.

The amino acid sequence 5A is the amino acid sequence of olfactory receptor 4 that is intrinsically expressed (inherently possessed) by *Aedes aegypti.*

The amino acid sequence 5B can be any amino acid sequence as long as the nitro compound responsiveness of a protein composed of a sequence containing the amino acid sequence 5B having the mutation 5a introduced (protein 5B) is not significantly reduced compared with the nitro compound responsiveness of a protein composed of a sequence containing the amino acid sequence 5A having the mutation 5a introduced (protein 5A). The nitro compound responsiveness of the protein 5B is, for example, 50% or more, preferably 60% or more, more preferably 70% or more, still more preferably 80% or more, and yet more preferably 90% or more, of the nitro compound responsiveness of the protein 5A taken as 100%. From this viewpoint, the identity of the amino acid sequence 5B to the amino acid sequence 5A is preferably at least 85%, more preferably at least 90%, still more preferably at least 95%, yet more preferably at least 97%, particularly preferably at least 99%, and less than 100%.

The amino acid sequence 5B is preferably the amino acid sequence described in (5B'): (5B') Amino acid sequence 5B' containing the amino acid sequence 5A with one or multiple amino acid mutations.

In the amino acid sequence 5B', "multiple" means, for example, 2 to 20, preferably 2 to 10, more preferably 2 to 5, still more preferably 2 to 3, and yet more preferably 2.

The amino acid mutation introduced into the amino acid sequence 5A contained in the amino acid sequence 5B is, for example, substitution, deletion, addition, or insertion, preferably substitution, and particularly more preferably conservative substitution.

The amino acid sequence 5B is preferably the amino acid sequence of an olfactory receptor derived from an insect. The olfactory receptor derived from an insect is as defined in the "2-1. Amino Acid Sequence 1C" section above.

The mutation 5a is substitution 5ax that is substitution of amino acid L178 in the amino acid sequence 5A or substitution 5ay that corresponds to the substitution 5ax in the amino acid sequence 5B. The phrase "substitution ... that corresponds to ..." is as defined in the "2-1. Amino Acid Sequence 1C" section above.

The amino acid after mutation of L178 or the amino acid after mutation of the amino acid that corresponds to L178 in the mutation 5a is preferably proline.

The amino acid sequence 5C may contain other amino acid mutations (e.g., substitution or conservative substitution) as long as the nitro compound responsiveness is not significantly impaired. In an embodiment, the nitro compound responsiveness can be further enhanced by introducing a specific mutation into the seven-transmembrane domain. The number of other amino acid mutations is, for example, 1 to 50, 1 to 20, 1 to 10, or 1 to 5.

The amino acid sequence 5C is, specifically, for example, the amino acid sequence of SEQ ID NO: 85 or an amino acid sequence with at least 80% identity (preferably at least 85%, more preferably at least 90%, still more preferably at least 95%, yet more preferably at least 97%, particularly preferably at least 99%, and less than 100% identity) to the amino acid sequence.

### 2-6. Protein

In one embodiment, the olfactory receptor protein of the present disclosure contains amino acid sequence B composed of amino acid sequence A containing a mutation,
the amino acid sequence A is in a corresponding wild-type olfactory receptor protein and represented by formula (1): φ₇X₁X₂Z₁X₃X₄Z₂Z₃φ₆Uφ₅φ₄φ₃φ₂φ₁X₅X₆ wherein X₁ to X₆ and Z₁ to Z₃ are amino acids derived from the amino acid sequence of a wild-type insect olfactory receptor, φ₁ represents a hydrophobic amino acid, φ₂ to φ₇ each independently represent an uncharged polar amino acid or a hydrophobic amino acid, and U represents an uncharged polar amino acid, and
the olfactory receptor protein can satisfy at least one condition selected from the group consisting of the following conditions 1 to 3:
   condition 1: in the amino acid sequence B, X₃ and/or X₄ is substituted, and X₃ is a branched-chain amino acid and/or X₄ is an uncharged polar amino acid,
   condition 2: if X₅ and X₆ in the amino acid sequence A are both a positively charged polar amino acid, X₆ in the amino acid sequence B is an amino acid other than a positively charged polar amino acid, and
   condition 3: if either X₁ or X₂ or both in the amino acid sequence A are a positively charged polar amino acid, in the amino acid sequence B, X₁ and/or X₂ is substituted, and X₁ is an uncharged polar amino acid and/or X₂ is a hydrophobic amino acid.

Due to the above, the nitro compound responsiveness can be improved. The phrase "corresponding wild-type olfactory receptor" means an olfactory receptor comprising any of the amino acid sequences 1A to 5A. The amino acid sequence A represented by formula (1) may be included in the seven-transmembrane domain.

The olfactory receptor protein of the present disclosure may have an amino acid sequence other than amino acid sequences 1C to 5C, such as a protein or peptide (e.g., a protein tag, a fluorescent protein, a photoprotein, or a signal sequence), added thereto, as long as the nitro compound responsiveness is not significantly impaired. Examples of protein tags include biotin, a His tag, a FLAG tag, a Halo tag, a MBP tag, a HA tag, a Myc tag, a V5 tag, a PA tag, and the like.

The olfactory receptor protein of the present disclosure may be chemically modified, as long as the nitro compound responsiveness is not significantly impaired.

The olfactory receptor protein of the present disclosure may have a carboxyl group (-COOH), carboxylate (-COO⁻), amide (-CONH₂), or ester (-COOR) at the C-terminus.

"R" in the ester is, for example, a C₁₋₆ alkyl group, such as methyl, ethyl, n-propyl, isopropyl, or n-butyl; a C₃₋₈ cycloalkyl group, such as cyclopentyl or cyclohexyl; a C₆₋₁₂ aryl group, such as phenyl or α-naphthyl; a phenyl-C₁₋₂ alkyl group, such as benzyl or phenethyl; a C₇₋₁₄ aralkyl group including an α-naphthyl-C₁₋₂ alkyl group, such as α-naphthylmethyl; a pivaloyloxymethyl group; or the like.

The olfactory receptor protein of the present disclosure may have an amidated or esterified carboxyl group (or carboxylate), which is not the carboxyl group at the C-terminus. As the ester in this case, for example, the above-mentioned C-terminal ester or the like is used.

Furthermore, the olfactory receptor protein of the present disclosure also includes those in which the amino group of the N-terminal amino acid residue is protected by a protecting group (e.g., a C₁₋₆ acyl group such as C₁₋₆ alkanoyl, such as a formyl group or an acetyl group); those in which the N-terminal glutamine residue that can be produced by cleavage in vivo is converted to pyroglutamic acid; those in which a substituent (e.g., -OH, -SH, an amino group, an imidazole group, an indole group, or a guanidino group) on an amino acid side chain in the molecule is protected by an appropriate protecting group (e.g., a C₁₋₆ acyl group such as C₁₋₆ alkanoyl, such as a formyl group or an acetyl group); conjugated proteins such as those called glycoproteins having a sugar chain bound thereto; and the like.

The olfactory receptor protein of the present disclosure may be in the form of a salt with an acid or a base. The salt is not particularly limited, and acid salts and basic salts can both be employed. Examples of acid salts include inorganic acid salts, such as hydrochloride, hydrobromide, sulfate, nitrate, and phosphate; organic acid salts, such as acetate, propionate, tartrate, fumarate, maleate, malate, citrate, methanesulfonate, and p-toluenesulfonate; amino acid salts, such as aspartate and glutamate; and the like. Examples of basic salts include alkali metal salts, such as sodium salt and potassium salt; alkaline earth metal salts, such as calcium salt and magnesium salt; and the like.

The olfactory receptor protein of the present disclosure may be in the form of a solvate. The solvent is not particularly limited, and examples include water, ethanol, glycerol, acetic acid, and the like.

The olfactory receptor protein of the present disclosure can be easily produced according to a known genetic engineering method. For example, the olfactory receptor protein of the present disclosure can be produced using PCR, restriction enzyme cleavage, a DNA ligation technique, an in vitro transcription/translation technique, a recombinant protein production technique, etc.

### 3. Polynucleotide, Cell, and Non-human Animal

In one embodiment, the present disclosure relates to a polynucleotide comprising a coding sequence of the olfactory receptor protein of the present disclosure (which may be referred to as "the polynucleotide of the present disclosure" in the present specification), a cell comprising the polynucleotide of the present disclosure (which may be referred to as "the cell of the present disclosure" in the present specification), and a non-human animal comprising the cell of the present disclosure (which may be referred to as "the non-human animal of the present disclosure" in the present specification). These are described below.

The coding sequence of the olfactory receptor protein of the present disclosure is not particularly limited as long as it is a polynucleotide comprising a base sequence encoding the olfactory receptor protein of the present disclosure.

In one embodiment, the polynucleotide of the present disclosure include an expression cassette for the olfactory receptor protein of the present disclosure.

The expression cassette for the olfactory receptor protein of the present disclosure is not particularly limited as long as it is a polynucleotide capable of expressing the olfactory receptor protein of the present disclosure in a cell. Typical examples of the expression cassette for the olfactory receptor protein of the present disclosure include a polynucleotide containing a promoter and the coding sequence of the olfactory receptor protein of the present disclosure placed under the control of the promoter.

The promoter contained in the expression cassette for the olfactory receptor protein of the present disclosure is not particularly limited, and can be appropriately selected depending on the target cell. For example, various pol II promoters can be used. Examples of pol II promoters include, but are not limited to, a CMV promoter, an EF1 promoter, an SV40 promoter, an MSCV promoter, and the like. Other examples of the promoter include a tryptophan promoter such as trc or tac, a lac promoter, a T7 promoter, a T5 promoter, a T3 promoter, an SP6 promoter, an arabinose-inducible promoter, a cold-shock promoter, a tetracycline-inducible promoter, and the like.

The expression cassette for the olfactory receptor protein of the present disclosure may contain other elements (e.g., a multiple cloning site (MCS), a drug resistance gene, an origin of replication, an enhancer sequence, a repressor sequence, an insulator sequence, a reporter protein (e.g., fluorescent protein) coding sequence, and a drug resistance gene coding sequence), if necessary.

The polynucleotide of the present disclosure may be in the form of a vector. An appropriate vector is selected according to the intended use (cloning, expression of protein) and in consideration of the kind of host cell. Examples of a vector for *Escherichia coli* as a host include M13 phage or a modified form thereof, λ phage or a modified form thereof, pBR322 or a modified form thereof (e.g., pB325, pAT153, and pUC8), and the like; examples of a vector for a yeast as a host include pYepSec1, pMFa, pYES2, pPIC3.5K, and the like; examples of a vector for an insect cell as a host include pAc, pVL, and the like; and examples of a vector for a mammalian cell as a host include pcDNA, pCDM8, pMT2PC, and the like.

The cell of the present disclosure is not particularly limited as long as it contains the polynucleotide of the present disclosure. Examples of the cell include colon bacteria such as *Escherichia coli* K12; *Bacillus* bacteria such as *Bacillus subtilis* MI114; yeasts such as *Saccharomyces cerevisiae* AH22; insect cells, such as *Spodoptera frugiperda-*derived Sf cell line, *Trichoplusia ni*-derived High Five cell line, and olfactory neurons; animal cells such as COS7 cells; and the like. Preferred examples of animal cells include mammal-derived cultured cells, such as COS7 cells, CHO cells, HEK293 cells, HEK293FT cells, Hela cells, PC12 cells, N1E-115 cells, SH-SY5Y cells, and the like.

From the viewpoint that the cell of the present disclosure can be used as is for the nitro compound detection described later, the cell of the present disclosure preferably contains a coding sequence for an insect olfactory receptor co-receptor. The insect olfactory receptor co-receptor is a membrane protein having a seven-transmembrane structure as in olfactory receptors; however, the insect olfactory receptor co-receptor itself does not recognize odorants and functions by forming a heterocomplex with an olfactory receptor. The olfactory receptor complex, a heterocomplex composed of an olfactory receptor and an olfactory receptor co-receptor, has ion channel activity that is activated by an odorant, allowing cations such as sodium ions (Na⁺) and calcium ions (Ca²⁺) to flow into cells when activated.

From the same viewpoint, the cell of the present disclosure preferably contains a coding sequence for a protein that develops color or emits light due to ions (e.g., calcium ions) flown into the cell upon response of an olfactory receptor. Examples of the protein include aequorin, yellow cameleon (YC), GCaMP, and the like. Alternatively, the cell of the present disclosure preferably contains a calcium ion-dependent fluorescent dye (e.g., Fura-2, Fluo-3, or Fluo-4).

From the same viewpoint, the cell of the present disclosure contains the olfactory receptor protein of the present disclosure, i.e., the olfactory receptor protein of the present disclosure is expressed in the cell of the present disclosure. In this case, the olfactory receptor protein of the present disclosure, which has a seven-transmembrane structure, is positioned as a membrane protein on the cell membrane.

The non-human animal of the present disclosure is not particularly limited as long as it comprises the cell of the present disclosure. Although there is no particular limitation, the non-human animal is preferably an insect, from the viewpoint of being suitable for use in the nitro compound detection described later. The insect is as described in the "2-1. Amino Acid Sequence 1C" section above. From the same viewpoint, it is also preferred that the cells of the present disclosure in the non-human animal of the present disclosure comprise olfactory neurons.

### 4. Use

The olfactory receptor protein of the present disclosure has the activity of allowing cations (sodium ions (Na⁺), calcium ions (Ca²⁺), or the like) to flow into cells in response to a nitro compound. The nitro compound can be detected by detecting the cations or by detecting behavioral changes based on olfactory neuron activation induced by the influx of the cations. Thus, the olfactory receptor protein of the present disclosure can be used as a nitro compound detection element.

In one embodiment, the present disclosure relates to a nitro compound detection element comprising the olfactory receptor protein of the present disclosure (the detection element of the present disclosure), and further relates to a nitro compound detection sensor that comprises a cell comprising the detection element of the present disclosure, a lipid bilayer (artificial cell membrane) comprising the detection element of the present disclosure, or a non-human animal comprising the cell (the detection sensor of the present disclosure).

The nitro compound is not particularly limited as long as it is a nitro group-containing compound. The nitro compound is preferably, for example, a compound having a structure in which a nitro group is directly linked to a benzene ring, a compound containing two or more nitro groups, or the like. Specific examples of the nitro compound include 2-nitrotoluene, 3-nitrotoluene, 4-nitrotoluene, 2,3-dinitrotoluene, 2,4-dinitrotoluene, 2,6-dinitrotoluene, 4-amino-2,6-dinitrotoluene, 3-trifluoromethyl-4-nitrophenol, nitroaniline, trinitrotoluene, trimethylenetrinitramine, pentaerythritol tetranitrate, picric acid, nitroglycerin, 2,3-dimethyl-2,3-dinitrobutane, and the like.

Chemical substance detection sensors using cells, lipid bilayers, or non-human animals are already known, and as their specific configurations, the configurations described in, for example, PTL 1, PTL 2, PTL 3, etc. can be used. The nitro compound detection sensor comprising the cell or lipid bilayer of the present disclosure comprises, for example, a container holding the cell or lipid bilayer of the present disclosure, a sensor that outputs a signal upon detection of a nitro compound response (e.g., light) in the cell of the present disclosure, and a determiner that detects a nitro compound based on the signal. The nitro compound detection sensor comprising the non-human animal of the present disclosure comprises, for example, a detector (e.g., a motion sensor, a vibration sensor, or a sound sensor) that detects movement of the non-human animal of the present disclosure.

### Examples

Embodiments of the present invention are described in more detail with reference to Examples. However, the present invention is not limited to these Examples.

In the following Examples,
OR stands for olfactory receptor (odorant receptor);
Orco stands for olfactory receptor co-receptor (odorant receptor co-receptor);
Ag stands for *Anopheles gambiae;* and
Aa stands for *Aedes aegypti.*

The wild-type olfactory receptor genes used in the following Examples are as follows.

**Table 1**

| Source organism name | Wild-type gene | Base sequence: SEQ ID NO. | Amino acid sequence: SEQ ID NO. | Accession No. |
|---|---|---|---|---|
| *Anopheles gambiae* | AgOR6 | SEQ ID NO: 8 | SEQ ID NO: 3 | XM_316227.4 |
| *Anopheles gambiae* | AgOR15 | SEQ ID NO: 9 | SEQ ID NO: 4 | XM_310073.1 |
| *Anopheles* | AgOR28 | SEQ ID NO: 6 | SEQ ID NO: 1 | XM_312203.1 |
| *gambiae* | | | | |
| *Aedes aegypti* | AgOR47 | SEQ ID NO: 7 | SEQ ID NO: 2 | XM_310064.1 |
| *Aedes aegypti* | AaOR4 type 1 | SEQ ID NO: 10 | SEQ ID NO: 5 | NH_001358193.1 |
| *Aedes aegypti* | AaOR4 type 2 | SEQ ID NO: 86 | SEQ ID NO: 85 | BK006140.1 |

### Test Example 1: Measurement of Nitro Compound Responsiveness of AgOR28

The response activity of olfactory receptors (wild-type AgOR28 and AgOR28 mutant) to nitro compounds was measured.

### Test Example 1-1. Preparation of Expression Plasmids

### Comparison Example 1: Preparation of Wild-type AgOR28 Expression Plasmid

Double-stranded DNA containing the coding sequence (base sequence: SEQ ID NO: 6) of wild-type AgOR28 (amino acid sequence: SEQ ID NO: 1) on one of the DNA strands was synthesized and introduced into pcDNA3.1 to obtain a wild-type AgOR28 expression plasmid (pcDNA3.1-AgOR28).

### Comparative Example 2: Preparation of Mutant AgOR28 (T25K mutation) Expression Plasmid

A plasmid (pcDNA3.1) containing a sequence (base sequence: SEQ ID NO: 12) encoding an AgOR28 mutant comprising the amino acid sequence of wild-type AgOR28 in which the 25th amino acid residue threonine was mutated to lysine (T25K mutation) (amino acid sequence: SEQ ID NO: 11) was prepared in the following manner.

PCR primers designed to convert the 25th amino acid residue threonine in the amino acid sequence of wild-type AgOR28 to lysine (CAGAGGCATGGCCAAGAAGATCCAGAACAGC (SEQ ID NO: 13) and GCTGTTCTGGATCTTCTTGGCCATGCCTCTG (SEQ ID NO: 14)) were synthesized. Using these primers, PCR amplification was performed using pcDNA3.1-AgOR28 as a template. A PCR reaction was performed using KOD -Plus- Neo (produced by Toyobo Co., Ltd.). The composition of the reaction solution was prepared according to the protocol attached to the KOD -Plus- Neo kit by using 1 × PCR buffer for KOD -Plus- Neo, 0.2 mM dNTPs, 1.5 mM MgSO_{4,} 0.2 µM primer, and 1 U KOD -Plus- Neo DNA polymerase and adjusting the obtained solution to a final volume of 50 µL. After pre-denaturation at 95°C for 30 seconds, a reaction cycle consisting of denaturation at 95°C for 30 seconds, annealing at 55°C for 1 minute, and elongation reaction at 68°C for 10 minutes was repeated 16 times. 0.4 µL of DpnI was added to 20 µL of the obtained PCR product, and the PCR product was treated at 37°C for 2 hours and then transformed into *E. coli* (DH5α). The transformed *E. coli* were seeded on ampicillin-supplemented LB plates and cultured at 37°C to form colonies. Two colonies were selected and cultured again in LB liquid medium. The plasmids harbored in the colonies were then sequenced to obtain a mutant AgOR28 (T25K mutation) expression plasmid.

### Comparative Example 3: Preparation of Mutant AgOR28 (P31T Mutation) Expression Plasmid

A plasmid (pcDNA3.1) containing a sequence (base sequence: SEQ ID NO: 16) encoding an AgOR28 mutant comprising the amino acid sequence of wild-type AgOR28 in which the 31st amino acid residue proline was mutated to threonine (P31T mutation) (amino acid sequence: SEQ ID NO: 15) was produced. Specifically, the plasmid was produced in the same manner as in Comparative Example 2 except that primer F: CAGATCCAGAACAGCACCATCGACCTGTACGTG (SEQ ID NO: 17) and primer R: CACGTACAGGTCGATGGTGCTGTTCTGGATCTG (SEQ ID NO: 18) were used as PCR primers.

### Comparative Example 4: Preparation of AgOR28 Mutant (T25K-P31T Double Mutation) Expression Plasmid

A plasmid (pcDNA3.1) containing a sequence (base sequence: SEQ ID NO: 20) encoding an AgOR28 mutant comprising the amino acid sequence of wild-type AgOR28 in which the 25th amino acid residue threonine was mutated to lysine and the 31st amino acid residue proline was mutated to threonine (T25K, P31T double mutation) (amino acid sequence: SEQ ID NO: 19) was prepared. Specifically, the plasmid was prepared in the same manner as in Comparative Example 3 except that the AgOR28 mutant (T25K mutation) expression plasmid (Comparative Example 2) was used as a template for PCR.

### Example 1: Preparation of AgOR28 Mutant (22a.a deletion) Expression Plasmid

A plasmid (pcDNA3.1) containing a sequence (base sequence: SEQ ID NO: 22) encoding an AgOR28 mutant comprising the amino acid sequence of wild-type AgOR28 in which 22 amino acids from position 52 to position 73 were sequentially deleted (22a.a deletion) (amino acid sequence: SEQ ID NO: 21) was produced. Specifically, as PCR primers, primer F1: TACCGAGCTCGGATCATGGCCAGACTGGTGCTG (SEQ ID NO: 23), primer R1: GATATCTGCAGAATT TTACTGCTGGTTGATGGTC (SEQ ID NO: 24), primer F2: GCCAGCCTGTGCGTGCCCCAGTTCACCTACCTGGTGGTGGACACCAAG (SEQ ID NO: 90), and primer R2: CACCAGGTAGGTGAACTGGGGCACGCACAGGCTGGCGATAGGGATG (SEQ ID NO: 91) were synthesized. Using wild-type AgOR28 as a template, PCR with primers F1 and R2 and PCR with primers F2 and R1 were performed. Each of the amplified products was purified by electrophoresis on an agarose gel. The purified products were mixed at a 1:1 molar ratio and incubated at 95°C for 5 minutes, and then allowed to stand at room temperature for 5 minutes. A reaction solution containing 10 µL of this mixture and adjusted to a final volume of 50 µL (1U KOD -Plus- Neo DNA polymerase (produced by Toyobo Co., Ltd.), 1.5 mM MgSO₄ (produced by Toyobo Co., Ltd.), 0.2 mM dNTPs Mix (produced by Toyobo Co., Ltd.), and 1 × PCR Buffer for KOD -Plus- Neo PCR (produced by Toyobo Co., Ltd.) was prepared. After 94°C for 2 minutes, a reaction cycle consisting of 95°C for 30 seconds, 63°C for 30 seconds, and 68°C for 1 minute and 50 seconds was performed twice. 5 µL of this reaction solution was isolated and PCR was performed using primers F1 and R1. The PCR reaction solution was prepared by using the following composition: 1 × PCR buffer for KOD -Plus-Neo, 0.2 mM dNTPs, 1.5 mM MgSO_{4,} 0.2 µM primer, and 1U KOD -Plus-Neo DNA polymerase and adjusting the solution to a final volume of 50 µL. After pre-denaturation at 94°C for 2 minutes, a reaction cycle consisting of denaturation at 98°C for 10 seconds, annealing at 63°C for 30 seconds, and an elongation reaction at 68°C for 1 minute and 50 seconds, was performed 30 times. The obtained PCR amplified fragment and pcDNA3.1 expression plasmid fragment cleaved by BamHI and EcoRI were subjected to an in-fusion reaction using an In-Fusion HD Cloning Kit (produced by Takara Bio, Inc.) according to the protocol attached to the In-Fusion HD Cloning Kit to link the fragments. After completion of the reaction, the In-Fusion reaction solution was used to transform the resulting fragment into *E. coli* (DH5α). The transformed *E. coli* was seeded on ampicillin-supplemented LB plates and cultured at 37°C to form colonies. Two colonies were selected and cultured again in LB liquid medium. The plasmids harbored in the colonies were then sequenced to obtain expression plasmids.

The amino acid sequence of wild-type AgOR28 was subjected to structure prediction (under default conditions) using TMpred (K. Hofmann, W. Stoffel, TMbase - a database of membrane spanning proteins segments, Biol. Chem. Hoppe-Seyler, 374 (1993), p. 166, https:// (embnet.vital-it.ch/software/TMPRED_form.html). The amino acid region from position 55 to position 84 was predicted to be the first extracellular loop. This indicates that the deletion region was the first extracellular loop.

### Example 2: Preparation of AgOR28 Mutant (T25K Mutation, 22a.a Deletion) Expression Plasmid

A plasmid (pcDNA3.1) containing a sequence (base sequence: SEQ ID NO: 26) encoding an AgOR28 mutant comprising the amino acid sequence of wild-type AgOR28 in which the 25th amino acid residue threonine was mutated to lysine, and 22 amino acids from position 52 to position 73 were sequentially deleted (T25K mutation, 22a.a deletion) (amino acid sequence: SEQ ID NO: 25) was prepared. Specifically, the plasmid was prepared in the same manner as in Comparative Example 2 except that the AgOR28 mutant (22a.a deletion) expression plasmid (Example 1) was used as a template for PCR.

### Example 3: Preparation of AgOR28 Mutant (P31T Mutation, 22a.a Deletion) Expression Plasmid

A plasmid (pcDNA3.1) containing a sequence (base sequence: SEQ ID NO: 28) encoding an AgOR28 mutant comprising the amino acid sequence of wild-type AgOR28 in which the 31st amino acid residue proline was mutated to threonine, and 22 amino acids from position 52 to position 73 were sequentially deleted (P31T mutation, 22a.a deletion) (amino acid sequence: SEQ ID NO: 27) was prepared. Specifically, the plasmid was prepared in the same manner as in Comparative Example 3 except that the AgOR28 mutant (22a.a deletion) expression plasmid (Example 1) was used as a template for PCR.

### Example 4: Preparation of AgOR28 Mutant (T25K-P31T Double Mutation, 22a.a Deletion) Expression plasmid

A plasmid (pcDNA3.1) containing a sequence (base sequence: SEQ ID NO: 30) encoding an AgOR28 mutant comprising the amino acid sequence of wild-type AgOR28 in which the 25th amino acid residue threonine was mutated to lysine, the 31st amino acid residue lysine was mutated to threonine, and 22 amino acids from position 52 to position 73 were sequentially deleted (T25K-P31T double mutation, 22a.a deletion) (amino acid sequence: SEQ ID NO: 29) was prepared. Specifically, the plasmid was prepared in the same manner as in Comparative Example 3 except that the mutant AgOR28 (T25K mutation, 22a.a deletion) expression plasmid (Example 2) was used as a template for PCR.

### Reference Example 1. Preparation of Chimeric Orco Expression Plasmids

A plasmid (pcDNA3.1) containing a sequence (base sequence: SEQ ID NO: 93) encoding fusion protein Dm(NT-TM4) AmOrco, which is a fusion protein of part of *Drosophila* co-receptor and part of *Apis* co-receptor, (amino acid sequence: SEQ ID NO: 92) was produced according to a previous report (JP2018-50556A). In Dm(NT-TM4) AmOrco, the amino acid sequence from the N-terminal region (NT) to the four-transmembrane domain (TM4) (an amino acid sequence from its N-terminal amino acid residue to the 234th amino acid residue) is derived from a *Drosophila* co-receptor (Dm(NT-TM4), whereas the amino acid sequence from the second intracellular loop to the C-terminal region is derived from an *Apis* co-receptor (Am(IC2-CT)).

### Test Example 1-2. Introduction of Expression Plasmid into Cells

HEK293FT cells (purchased from Invitrogen, Inc.) were seeded in 10-cm petri dishes at 3 × 10⁶ cells/petri dish and cultured in DMEM medium containing 10% FBS (produced by Nacalai Tesque, Inc.) at 37°C and 5% CO₂ for about 24 hours. 1.5 µg of any one of the olfactory receptor expression plasmids prepared, 3.0 µg of an olfactory receptor co-receptor expression plasmid, and 8 µg of GFP-aequorin (GAP) expression plasmid were mixed with 12.5 µL of a Plus reagent and 31.25 µL of Lipofectamine LTX (produced by Invitrogen, Inc.), and the resulting mixture was maintained for 10 minutes. This mixture was then transfected into the above cells. Four hours after the start of transfection, the cells were seeded in 96-well plates at 9 × 10⁴ cells/well and cultured in DMEM medium (produced by Nacalai Tesque, Inc.) containing 10% FBS at 37°C and 5% CO₂ for about 24 hours. A transformed cell in which the olfactory receptor expression plasmid, olfactory receptor co-receptor expression plasmid, and GAP expression plasmid were transiently introduced was thereby obtained.

### Test Example 1-3. Activity Measurement

When bound to calcium ions, GFP-aequorin (GAP) is activated and emits green fluorescence in the presence of a substrate, such as coelenterazine. Accordingly, an increase of intracellular calcium ion concentration in GAP-expressing cells directly appears as an increase in the amount of fluorescence. Therefore, by adding a test substance, whether the olfactory receptor complex functions as an ion channel can be determined from changes in the amount of fluorescence.

The culture medium of the transformed cells was removed and replaced with Assay buffer (Hanks-HEPES (20 mM pH 7.4) containing 0.5 µM Coelenterazine-h (produced by Promega Corporation) and 0.3% BSA) and allowed to stand at room temperature for another 4 hours. Subsequently, using a FlexStation 3 (produced by Molecular Devices, LLC), the amount of luminescence of the cells was determined by adding a test substance corresponding to the olfactory receptor to the cell culture medium.

In this test example, 2,3-dinitrotoluene, 2,6-dinitrotoluene, or toluene was used as a test substance.

Fig. 1 shows the results. *Anopheles* olfactory receptor 28 containing 22a.a deletion was found to show nitrogen compound responsiveness, but no responsiveness to non-nitro compounds. In contrast, *Anopheles* olfactory receptor 28 not containing 22a.a deletion did not show nitro compound responsiveness.

### Test Example 2: Measurement of Nitro Compound Responsiveness of AgOR47

The response activity of olfactory receptors (wild-type AgOR47 and AgOR47 mutant) to nitro compounds was determined.

### Test Example 2-1: Preparation of Expression Plasmid

### Comparison Example 5: Production of Wild-type AgOR47 Expression Plasmid

Double-stranded DNA containing the coding sequence (SEQ ID NO: 7) of wild-type AgOR47 (amino acid sequence: SEQ ID NO: 2) on one of the DNA strands was synthesized and introduced into pcDNA3.1 to obtain a wild-type AgOR47 expression plasmid (pcDNA3.1-AgOR47).

### Comparison Example 6: Preparation of Mutant AgOR47 (L71F mutation) Expression Plasmid

A plasmid (pcDNA3.1) containing a sequence (base sequence: SEQ ID NO: 36) encoding an AgOR47 mutant comprising the amino acid sequence of wild-type AgOR47 in which the 71st amino acid residue leucine was mutated to phenylalanine (L71F mutation) (amino acid sequence: SEQ ID NO: 35) was produced in the following manner.

PCR primers designed to convert the 71st amino acid residue leucine in the amino acid sequence of wild-type AgOR47 to phenylalanine (primer F: CTATCGCCGAGGGCATGTTCAGCTTCAATACCACC (SEQ ID NO: 37) and primer R: GGTGGTATTGAAGCTGAACATGCCCTCGGCGATAG (SEQ ID NO: 38)) were synthesized. Using these primers, PCR amplification was performed using pcDNA3.1-AgOR47 as a template (the reaction procedure and conditions were according to the standard method). The PCR reaction solution after amplification was digested with DpnI and then transformed into *E. coli* (DH5α). The transformed *E. coli* were seeded on ampicillin-supplemented LB plates and cultured at 37°C to form colonies. Two colonies were selected and cultured again in LB liquid medium. The plasmids harbored in the colonies were then sequenced to obtain an AgOR47 mutant (L71F mutation) expression plasmid.

### Comparative Example 7: Preparation of AgOR47 Mutant (I134V mutation) Expression Plasmid

A plasmid (pcDNA3.1) containing a sequence (base sequence: SEQ ID NO: 40) encoding an AgOR47 mutant comprising the amino acid sequence of wild-type AgOR47 in which the 134th amino acid residue isoleucine was mutated to valine (I134V mutation) (amino acid sequence: SEQ ID NO: 39) was prepared. Specifically, the plasmid was prepared in the same manner as in Comparative Example 6 except that primer F: CATCTTCACCAACGGCGTCGTGTTCGCCATGAC (SEQ ID NO: 41) and primer R: GGTCATGGCGAACACGACGCCGTTGGTGAAGATG (SEQ ID NO: 42) were used as PCR primers.

### Comparative Example 8: Preparation of AgOR47 Mutant (S140G Mutation) Expression Plasmid

A plasmid (pcDNA3.1) containing a sequence (base sequence: SEQ ID NO: 44) encoding an AgOR47 mutant comprising the amino acid sequence of wild-type AgOR47 in which the 140th amino acid residue serine was mutated to glycine (S140G mutation) (amino acid sequence: SEQ ID NO: 43) was prepared. Specifically, the plasmid was prepared in the same manner as in Comparative Example 6 except that primers F: GTGTTCGCCATGACCGGCTCTACAATCGCCGG (SEQ ID NO: 45) and primer R: CCGGCGATTGTAGAGCCGGTCATGGCGAACAC (SEQ ID NO: 46) were used as PCR primers.

### Comparative Example 9: Preparation of Mutant AgOR47 (F147L Mutation) Expression Plasmid

A plasmid (pcDNA3.1) containing a sequence (base sequence: SEQ ID NO: 48) encoding an AgOR47 mutant comprising the amino acid sequence of wild-type AgOR47 in which the 147th amino acid residue phenylalanine was mutated to leucine (F147L mutation)(amino acid sequence: SEQ ID NO: 47) was prepared. Specifically, the plasmid was prepared in the same manner as in Comparative Example 6 except that primer F: CAATCGCCGGCATGTTCTACACCTACTACACC (SEQ ID NO: 49) and primer R: GGTGTAGTAGGTGTAGAACATGCCGGCGATTG (SEQ ID NO: 50) were used as PCR primers.

### Comparative Example 10: Preparation of AgOR47 Mutant (D5E Mutation) Expression Plasmid

A plasmid (pcDNA3.1) containing a sequence (base sequence: SEQ ID NO: 52) encoding an AgOR47 mutant comprising the amino acid sequence of wild-type AgOR47 in which the fifth amino acid residue aspartic acid was mutated to glutamic acid (D5E mutation) (amino acid sequence: SEQ ID NO: 51) was prepared. Specifically, the plasmid was prepared in the same manner as in Comparative Example 6 except that primer F: ATGGTTGTGTTCGAACCACTGGACGACCC (SEQ ID NO: 53) and primer R: GGGTCGTCCAGTGGTTCGAACACAACCAT (SEQ ID NO: 54) were used as PCR primers.

### Comparative Example 11: Preparation of Mutant AgOR47 (K235Q Mutation) Expression Plasmid

A plasmid (pcDNA3.1) containing a sequence (base sequence: SEQ ID NO: 56) encoding an AgOR47 mutant comprising the amino acid sequence of wild-type AgOR47 in which the 235th amino acid residue lysine was mutated to glutamine (K235Q mutation) (amino acid sequence: SEQ ID NO: 55) was prepared. Specifically, the primer was prepared in the same manner as in Comparative Example 6 except that primer F: GATCGGACCCGTTGACCAGTATACTGCAGAGCTG (SEQ ID NO: 57) and primer R: CAGCTCTGCAGTATACTGGTCAACGGGTCCGATC (SEQ ID NO: 58) were used as PCR primers.

### Comparative Example 12: Preparation of AgOR47 Mutant (N241S Mutation) Expression Plasmid

A plasmid (pcDNA3.1) containing a sequence (base sequence: SEQ ID NO: 60) encoding an AgOR47 mutant comprising the amino acid sequence of wild-type AgOR47 in which the 241st amino acid residue asparagine was mutated to serine (N241S mutation) (amino acid sequence: SEQ ID NO: 59) was prepared. Specifically, the plasmid was prepared in the same manner as in Comparative Example 6 except that primer F: GTATACTGCAGAGCTGAGTGAAATTATCGAACTTCAC (SEQ ID NO: 61) and primer R: GTGAAGTTCGATAATTTCACTCAGCTCTGCAGTATAC (SEQ ID NO: 62) were used as PCR primers.

### Example 5: Production of AgOR47 Mutant (L60P Mutation) Expression Plasmid

A plasmid (pcDNA3.1) containing a sequence (base sequence: SEQ ID NO: 32) encoding an AgOR47 mutant comprising the amino acid sequence of wild-type AgOR47 in which the 60th amino acid residue leucine was mutated to proline (L60P mutation) (amino acid sequence: SEQ ID NO: 31) was prepared. Specifically, the plasmid was prepared in the same manner as in Comparative Example 6 except that primer F: CTACGAGACAATCCCGCAGTGCTTCCGGTC (SEQ ID NO: 33) and primer R: GACCGGAAGCACTGCGGGATTGTCTCGTAG (SEQ ID NO: 34)) were used as PCR primers.

### Example 6: Preparation of Mutant AgOR47 (M138I Mutation) Expression Plasmid

A plasmid (pcDNA3.1) containing a sequence (base sequence: SEQ ID NO: 64) encoding an AgOR47 mutant comprising the amino acid sequence of wild-type AgOR47 in which the 138th amino acid residue methionine was mutated to isoleucine (M138I mutation) (amino acid sequence: SEQ ID NO: 63) was prepared. Specifically, the plasmid was produced in the same manner as in Comparative Example 6 except that primer F: GGCGTCGTGTTCGCCATCACCGGCTCTACAATC (SEQ ID NO: 65) and primer R: GATTGTAGAGCCGGTGATGGCGAACACGACGCC (SEQ ID NO: 66) were used as PCR primers.

### Example 7: Preparation of AgOR47 Mutant (A152T Mutation) Expression Plasmid

A plasmid (pcDNA3.1) containing a sequence (base sequence: SEQ ID NO: 68) encoding an AgOR47 mutant comprising the amino acid sequence of wild-type AgOR47 in which the 152nd amino acid residue alanine was mutated to threonine (A152T mutation) (amino acid sequence: SEQ ID NO: 67) was prepared. Specifically, the plasmid was prepared in the same manner as in Comparative Example 6 except that primer F: GGAATGTTTTACACCTACTACACGAAAGATTCGGAATATTCC (SEQ ID NO: 69) and primer R: GGAATATTCCGAATCTTTCGTGTAGTAGGTGTAAAACATTCC (SEQ ID NO: 70) were used as PCR primers.

### Example 8: Preparation of AgOR47 Mutant (L60P-A152T Double Mutation) Expression Plasmid

A plasmid (pcDNA3.1) containing a sequence (base sequence: SEQ ID NO: 72) encoding an AgOR47 mutant comprising the amino acid sequence of wild-type AgOR47 containing an L60P-A152T double mutation (L60P--A152T double mutation) (amino acid sequence: SEQ ID NO: 71) was prepared. Specifically, the expression plasmid was prepared in the same manner as in Example 7 except that the AgOR47 mutant (L60P mutation) expression plasmid (Example 5) was first used as a template for PCR.

### Example 9: Preparation of AgOR47 Mutant (M138I-A152T Double Mutation) Expression Plasmid

A plasmid (pcDNA3.1) containing a sequence (base sequence: SEQ ID NO: 74) encoding an AgOR47 mutant comprising the amino acid sequence of wild-type AgOR47 containing an M138I-A152T double mutation (M138I-A152T double mutation) (amino acid sequence: SEQ ID NO: 73) was prepared. Specifically, the plasmid was prepared in the same manner as in Example 7 except that the AgOR47 mutant (M138I mutation) expression plasmid (Example 6) was used as a template for PCR.

### Example 10: Preparation of Mutant AgOR47 (L60P-M138I-A152T Triple Mutation) Expression Plasmid

A plasmid (pcDNA3.1) containing a sequence (base sequence: SEQ ID NO: 76) encoding an AgOR47 mutant comprising the amino acid sequence of wild-type AgOR47 containing an L60P-M138I-A152T triple mutation (L60P-M138I-A152T triple mutation) (amino acid sequence: SEQ ID NO: 75) was prepared. Specifically, the plasmid was prepared in the same manner as in Example 6 except that the AgOR47 mutant (L60P-A152T double mutation) expression plasmid (Example 8) was used as a template for PCR.

### Example 11: Preparation of AgOR47 Mutant (L60P-L71F-I134V-S140G-F147L Quintuple Mutation) Expression Plasmid

A plasmid (pcDNA3.1) containing a sequence (base sequence: SEQ ID NO: 109) encoding an AgOR47 mutant comprising the amino acid sequence of wild-type AgOr47 containing an L60P-L71F-I134V-S140G-F147L quintuple mutation (L60P-L71F-I134V-S140G-F147L quintuple mutation) (amino acid sequence: SEQ ID NO: 108) was prepared. Specifically, using the AgOR47 mutant (L60P heavy mutation) expression plasmid (Example 5) as a template for PCR, mutations were sequentially introduced using the primers used in the above Comparative Example.

### Example 12: Preparation of AgOR47 Mutant (L60P-L71F-I134V-M138IS140G-F147L-A152T Septuple Mutation) Expression Plasmid

A plasmid (pcDNA3.1) containing a sequence (base sequence: SEQ ID NO: 78) encoding an AgOR47 mutant containing an L60P-L71F-I134V-M138I-S140G-F147L-A152T septuple mutation (L60P-L71F-I134V-M138I-S140G-F147L-A152T septuple mutation) (amino acid sequence: SEQ ID NO: 77) was prepared. Specifically, first using the AgOR47 mutant (L60P-L71F-I134V-S140G-F147L quintuple mutation) expression plasmid (Example 11) as a template for PCR and using primers used in the above Example and Comparative Example, mutations were sequentially introduced to produce the plasmid.

### Example 13: Preparation of AgOR47 Mutant (L60P-L71F-I134V-M138IS140G-F147L Sextuple Mutation) Expression Plasmid

A plasmid (pcDNA3.1) containing a sequence (base sequence: SEQ ID NO: 80) encoding an AgOR47 mutant comprising the amino acid sequence of wild-type AgOR47 containing an L60P-L71F-I134V-M138I-S140G-F147L sextuple mutation (L60P-L71F-I134V-M138IS140G-F147L sextuple mutation) (amino acid sequence: SEQ ID NO: 79) was prepared. Specifically, using the AgOR47 mutant (L60P-L71F-I134V-S140G-F147L quintuple mutation) expression plasmid (Example 11) as a template for PCR, the mutation was introduced using the primers used in the above Example.

### Test Example 2-2: Introduction of Expression Plasmid into Cells and Activity Measurement

The expression plasmid was introduced into cells in the same manner as in Test Example 1-2, and the activity was measured in the same manner as in Test Example 1-3. In this test example, 4-amino-2,6-dinitrotoluene, 3-trifluoromethyl-4-nitrophenol, 2,3-dinitrotoluene, 2,6-dinitrotoluene, or 2,3-dimethyl-2,3-dinitrobutane was used as a test substance.

Figs. 2 to 6 show the results. *Anopheles* olfactory receptor 47 containing a substitution of at least one amino acid selected from the group consisting of A61, M138, and A152 was found to have nitrogen compound responsiveness, whereas *Anopheles* olfactory receptor 47 not containing this deletion showed no nitrogen compound responsiveness.

### Test Example 3: Measurement of Nitro Compound Responsiveness of AgOR6

The response activity of olfactory receptors (wild-type AgOR6 and AgOR6 mutant) to a nitro compound was determined.

### Test Example 3-1 Preparation of Expression Plasmid

### Comparison Example 13: Production of Wild-type AgOR6 Expression Plasmid

Double-stranded DNA containing the coding sequence (SEQ ID NO: 8) of wild-type AgOR6 (amino acid sequence: SEQ ID NO: 3) on one of the DNA strands was synthesized and introduced into pcDNA3.1 to obtain a wild-type AgOR6 expression plasmid (pcDNA3.1-AgOR6).

### Example 14: Production of AgOR6 Mutant Expression Plasmid

A plasmid (pcDNA3.1) containing a sequence (base sequence: SEQ ID NO: 82) encoding an AgOR6 mutant comprising the amino acid sequence of wild-type AgOR6 in which the 114th amino acid residue serine was mutated to alanine, the 120th amino acid residue alanine was mutated to glutamic acid, the 162nd amino acid residue histidine was mutated to tyrosine, the 186th amino acid residue valine was mutated to alanine, the 352nd amino acid residue valine was mutated to methionine, the 368th amino acid residue serine was mutated to proline, a region consisting of 79 amino acids was substituted (including a replacement of 11 charged amino acids in the region from the third extracellular loop to the third intracellular loop with uncharged amino acids) (amino acid sequence: SEQ ID NO: 81) was prepared. Specifically, double-stranded DNA containing the coding sequence (SEQ ID NO: 82) of the AgOR6 mutant (amino acid sequence: SEQ NO: 81) on one of the DNA strands was synthesized and introduced into pcDNA3.1 to obtain an AgOR6 mutant expression plasmid (pcDNA3.1-AgOR6).

### Test Example 3-2: Introduction of Expression Plasmid into Cells and Activity Measurement

The expression plasmid was introduced into cells in the same manner as in Test Example 1-2, and the activity was measured in the same manner as in Test Example 1-3. In this test example, 2-nitroaniline was used as a test substance.

Fig. 7 shows the results. Mutant *Anopheles* olfactory receptor 6 was found to show nitro compound responsiveness, whereas wild-type *Anopheles* olfactory receptor 6 showed no nitro compound responsiveness.

### Test Example 4. Measurement of Nitro Compound Responsiveness of AgOR15

The response activity of olfactory receptors (wild-type AgOR15 and AgOR15 mutant) to nitro compounds was measured.

### Test Example 4-1: Preparation of Expression Plasmid

### Comparative Example 14: Preparation of Wild-type AgOR15 Expression Plasmids

Double-stranded DNA containing the coding sequence (base sequence: SEQ ID NO: 9) of wild-type AgOR15 (amino acid sequence: SEQ ID NO: 4) on one of the DNA strands was synthesized and introduced into pcDNA3.1 to obtain a wild-type AgOR15 expression plasmid (pcDNA3.1-AgOR15).

### Example 15: Preparation of Mutant AgOR15 Expression Plasmid

A plasmid (pcDNA3.1) containing a sequence (base sequence: SEQ ID NO: 84) encoding an AgOR15 mutant comprising the amino acid sequence of wild-type AgOR15 in which the 61st amino acid residue methionine was mutated to threonine, the 104th amino acid residue cysteine was mutated to arginine, the 112th amino acid residue arginine was mutated to glutamine, the 181st amino acid residue glycine was mutated to alanine, the 189th amino acid residue valine was mutated to isoleucine, the 327th amino acid residue isoleucine was mutated to valine (amino acid sequence: SEQ ID NO: 83) was prepared. Specifically, using the wild-type AgOR15 expression plasmid as a template, the M61T-C104R-R112Q-G181A-V189I-I327V sextuple mutant was produced using the following primers.
Primer set for M61T mutagenesis
   CCCGACCTTGAGATAACGATCATTGGCACCGCTG (base sequence: SEQ ID NO: 94)
   and CAGCGGTGCCAATGATCGTTATCTCAAGGTCGGG (base sequence: SEQ ID NO: 95)
Primer set for C104R mutagenesis
   CCCAAACGGTTATCCGTGCGTCACCTCCCGCG (base sequence: SEQ ID NO: 96)
   and CGCGGGAGGTGACGCACGGATAACCGTTTGGG (base sequence: SEQ ID NO: 97)
Primer set for R112Q mutagenesis
   CCTCCCGCGGTGGTCCAGCATTTGACGACCCAG (base sequence: SEQ ID NO: 98)
   and CTGGGTCGTCAAATGCTGGACCACCGCGGGAGG (base sequence: SEQ ID NO: 99)
Primer set for G181A mutagenesis
   CAGACTCGCACCTCGGCTACGCACTACCTGATC (base sequence: SEQ ID NO: 100)
   and GATCAGGTAGTGCGTAGCCGAGGTGCGAGTCTG (base sequence: SEQ ID NO: 101)
Primer set for V189I mutagenesis
   CTACCTGATCTTCGGTATCGTCATGACGCCTACC (base sequence: SEQ ID NO: 102) and GGTAGGCGTCATGACGATACCGAAGATCAGGTAG (base sequence: SEQ ID NO: 103)
Primer set for I327V mutagenesis
   CGTGTAGCACATACCGTTTACGAAAGTGGCTGG (base sequence: SEQ ID NO: 104)
   and CCAGCCACTTTCGTAAACGGTATGTGCTACACG (base sequence: SEQ ID NO: 105)

### Test Example 4-2: Introduction of Expression Plasmid into Cells and Activity Measurement

The expression plasmid was introduced into cells in the same manner as in Test Example 1-2, and the activity was measured in the same manner as in Test Example 1-3. In this test example, 2-nitroaniline was used as a test substance.

Fig. 8 shows the results. Mutant *Anopheles* olfactory receptor 15 was found to show nitrogen compound responsiveness, whereas wild-type *Anopheles* olfactory receptor 15 did not show nitrogen compound responsiveness.

### Test Example 5: Measurement of Nitro Compound Responsiveness of AaOR4

The response activity of olfactory receptors (two types of wild-type AaOR4) to nitro compounds was measured.

### Test Example 5-1. Preparation of Expression Plasmids

### Comparison Example 15: Preparation of Wild-type AaOR4 Type 1 Expression Plasmid

Double-stranded DNA containing the coding sequence (SEQ ID NO: 10) of wild-type AaOR4 type 1 (amino acid sequence: SEQ ID NO: 5) on one of the DNA strands was synthesized and introduced into pcDNA3.1 to obtain a wild-type AaOR4 type 1 expression plasmid (pcDNA3.1-AaOR4 type 1).

### Example 16: Preparation of Wild-type AaOR4 Type 2 Expression Plasmid

A plasmid (pcDNA3.1) containing the coding sequence (base sequence: SEQ ID NO: 86) of wild-type AaOR4 type 2 comprising the amino acid sequence of wild-type AaOR4 type 1 in which the 178th amino acid residue leucine was mutated to proline (amino acid sequence: SEQ ID NO: 85) was prepared. Specifically, the plasmid was prepared in the following manner.

PCR primers designed to convert the 178th amino acid residue leucine in the amino acid sequence of wild-type AaOR4 to proline (GGGTCAAGCTATTTCCGTACGTGATTTGGTTC (SEQ ID NO: 106) and GAACCAAATCACGTACGGAAATAGCTTGACCC (SEQ ID NO: 107)) were synthesized. Using these primers, PCR amplification was performed using pcDNA3.1-AaOR4 type 1 as a template (the reaction procedure and conditions were according to the standard method). The amplified PCR reaction solution was digested with DpnI and then transformed into *E. coli* (DH5α). The transformed *E. coli* were seeded on ampicillin-supplemented plates and cultured at 37°C to form colonies. Two colonies were selected and cultured again in LB liquid medium. The plasmids harbored in the colonies were then sequenced to obtain a wild-type AaOR4 type 2 (L178P mutation) expression plasmid.

### Test Example 5-2: Introduction of Expression Plasmid into Cells and Activity Measurement Test

The expression plasmid was introduced into cells in the same manner as in Example 1-2, and the activity was measured in the same manner as in Example 1-3. In this test example, 2,4-dinitrotoluene, 4-nitrotoluene, or toluene was used as a test substance.

Fig. 9 shows the results. Wild-type AaOR4 type 2 was found to show nitrogen compound responsiveness, whereas wild-type AaOR4 type 1 did not show nitrogen compound responsiveness.

### Test Example 6: Comparison with the Prior Art

Cells transfected with mouse olfactory receptor Olfr256_17 are known to respond to nitro compounds. Such cells are known to be producible by introducing plasmids encoding mouse olfactory receptor Olfr256_17, G protein (Gα15_olf), receptor transport protein (RTP1S), and GAP. Accordingly, in this test example, such cells (mouse olfactory receptor-expressing cells) were prepared and compared in terms of nitrogen compound responsiveness with cells obtained in one embodiment of the present invention (cells in which the AgOR28 mutant (T25K mutation, 22a.a deletion) (Example 2) was introduced (Test Examples 1-2)).

First, an expression plasmid (rho-myc-mOlfr256_17/pcDNA3.1) for mouse olfactory receptor Olfr256_17 having a rho tag and a myc tag added to the N-terminus (amino acid sequence: SEQ ID NO: 87), an expression plasmid for Gα15_olf (amino acid sequence: SEQ ID NO: 88) (Ga15_olf/pBK-CMV), and an expression plasmid (mRTP1S/pcDNA3.1) for RTPS (amino acid sequence: SEQ ID NO: 89) were prepared according to usual methods.

HEK293FT cells (purchased from Invitrogen Corporation) were seeded in 10-cm petri dishes at 3 × 10⁶ cells/petri dish and cultured in DMEM medium containing 10% FBS (produced by Nacalai Tesque, Inc.) at 37°C and 5% CO₂ for about 24 hours. The produced Ga15_olf/pBK-CMV (1 µg), rho-myc-mO1fr256_17/pcDNA3.1 (2.5 µg), mRTP1S/pcDNA3.1 (1 µg), and GFP-Aequorin/pcDNA3.1 (8 µg) were mixed with 12.5 µL of a Plus reagent and 31.25 µL of Lipofectamine LTX (produced by Invitrogen Corporation) and allowed to stand for 10 minutes. The mixture was then transfected into the above cells. Four hours after the start of transfection, the cells were seeded in 96-well plates at 9 × 10⁴ cells/well and cultured in DMEM medium containing 10% FBS (produced by Nacalai Tesque, Inc.) at 37°C and 5% CO₂ for about 24 hours. Transformed cells transiently transfected with the mouse olfactory receptor Olfr256_17, G protein, and receptor transporter protein expression plasmid, and GAP expression plasmid (mouse olfactory receptor-expressing cells) were thereby obtained.

Using the cells transduced with AgOR28 mutant (T25K mutation, 22a.a deletion) (Example 2) (Test Example 1-2) and the mouse olfactory receptor-expressing cells obtained above, the activity was measured in the same manner as in Test Example 1-3. In this test example, 3-nitrotoluene was used as a test substance.

Fig. 10 shows the results. The mutant AgOR28 (T25K mutation, 22a.a deletion) was found to be capable of detecting the nitro compound with higher sensitivity and higher activity than the mouse olfactory receptor.

### Test Example 7. Introduction of Amino Acid Mutations into the Seven-transmembrane Domain

An expression plasmid for an AgOR28 mutant further containing amino acid substitutions (S368A, Q376A, R380A, S382L, S385A) in the seven-transmembrane domain of the AgOR28 mutant (T25K-P31T double mutant, 22a.a deletion) (Example 4) (T25K-P31T-S368A-Q376A-R380A-S382L-S385A septuple mutation, 22a.a deletion) was prepared in the same manner as in Example 4 or the like by using a PCR-based mutagenesis technique. The expression plasmid was introduced into the cells in the same manner as in Test Example 1-2, and the activity was measured in the same manner as in Test Example 1-3. In this test example, 2,3-dinitrotoluene was used as a test substance.

Fig. 11 shows the results. Mutagenesis in the seven-transmembrane domain was found to enhance nitro compound responsiveness.

## Claims

1. A nitro compound detection element comprising an olfactory receptor protein, the olfactory receptor protein comprising
(1) amino acid sequence 1C composed of amino acid sequence 1A of SEQ ID NO: 1 or amino acid sequence 1B with at least 80% identity to the amino acid sequence 1A, the amino acid sequence 1A and the amino acid sequence 1B each containing a mutation, wherein the amino acid sequence 1C contains mutation 1a, and the mutation 1a is deletion of a partial or entire region of the first extracellular loop;
(2) amino acid sequence 2C composed of amino acid sequence 2A of SEQ ID NO: 2 or amino acid sequence 2B with at least 80% identity to the amino acid sequence 2A, the amino acid sequence 2A and the amino acid sequence 2B each containing a mutation,
wherein the amino acid sequence 2C contains mutation 2a, and the mutation 2a is substitution 2ax or substitution 2ay,
the substitution 2ax is substitution of at least one amino acid selected from the group consisting of L60, M138, and A152 in the amino acid sequence 2A, and
the substitution 2ay corresponds to the substitution 2ax in the amino acid sequence 2B;
(3) amino acid sequence 3C composed of amino acid sequence 3A of SEQ ID NO: 3 or amino acid sequence 3B with at least 80% identity to the amino acid sequence 3A, the amino acid sequence 3A and the amino acid sequence 3B each containing a mutation,
wherein the amino acid sequence 3C contains mutation 3a and mutation 3b,
the mutation 3a is substitution 3ax or substitution 3ay,
the substitution 3ax is substitution of amino acids S114, A120, H162, V186, V352, and S368 in the amino acid sequence 3A, and the substitution 3ay corresponds to the substitution 3ax in the amino acid sequence 3B,
the mutation 3b is substitution of a charged amino acid into an uncharged amino acid in a region from the third extracellular loop to the third intracellular loop;
(4) amino acid sequence 4C composed of amino acid sequence 4A of SEQ ID NO: 4 or amino acid sequence 4B with at least 80% identity to the amino acid sequence 4A, the amino acid sequence 4A and the amino acid sequence 4B each containing a mutation,
wherein the amino acid sequence 4C contains mutation 4a, and the mutation 4a is substitution 4ax or substitution 4ay,
the substitution 4ax is substitution of amino acids M61, C104, R112, G181, V189, and I327 in the amino acid sequence 4A, and the substitution 4ay corresponds to the substitution 4ax in the amino acid sequence 4B; or
(5) amino acid sequence 5C composed of amino acid sequence 5A of SEQ ID NO: 5 or amino acid sequence 5B with at least 80% identity to the amino acid sequence 5A, the amino acid sequence 5A and the amino acid sequence 5B each containing a mutation,
wherein the amino acid sequence 5C contains mutation 5a, and the mutation 5a is substitution 5ax or substitution 5ay,
the substitution 5ax is substitution of amino acid L178 in the amino acid sequence 5A, and the substitution Say corresponds to the substitution 5ax in the amino acid sequence 5B.

2. The nitro compound detection element according to claim 1, wherein
in the mutation 1a, the deleted region is a region of 5 to 30 amino acids from the amino acid at the N-terminus;
in the mutation 2a, the amino acid after mutation of L60 is proline, the amino acid after mutation of M138 is a branched-chain amino acid, and/or the amino acid after mutation of A152 is a hydrophilic neutral amino acid;
in the mutation 3a, the amino acid after mutation of S114 is an aliphatic amino acid, the amino acid after mutation of A120 is an acidic amino acid, the amino acid after mutation of H162 is an aromatic amino acid, the amino acid after mutation of V186 is an aliphatic amino acid, the amino acid after mutation of V352 is a sulfur-containing amino acid, and the amino acid after mutation of S368 is proline;
in the mutation 3b, the number of substitutions of a charged amino acid into an uncharged amino acid is 6 to 18;
in the mutation 4a, the amino acid after mutation of M61 is a hydrophilic neutral amino acid, the amino acid after mutation of C104 is a basic amino acid, the amino acid after mutation of R112 is a hydrophilic neutral amino acid, the amino acid after mutation of G181 is an aliphatic amino acid, the amino acid after mutation of V189 is a branched-chain amino acid, and the amino acid after mutation of I327 is a branched-chain amino acid; and
in the mutation 5a, the amino acid after mutation of L178 is proline.

3. The nitro compound detection element according to claim 1 or 2, wherein
in the mutation 1a, the deleted region is a region of 15 to 25 amino acids from the amino acid at the N-terminus;
in the mutation 2a, the amino acid after mutation of L60 is proline, the amino acid after mutation of M138 is isoleucine, and/or the amino acid after mutation of A152 is threonine;
in the mutation 3a, the amino acid after mutation of S114 is alanine, the amino acid after mutation of A120 is glutamic acid, the amino acid after mutation of H162 is tyrosine, the amino acid after mutation of V186 is alanine, the amino acid after mutation of V352 is methionine, and the amino acid after mutation of S368 is proline;
in the mutation 3b, the number of substitutions of a charged amino acid into an uncharged amino acid is 8 to 14;
in the mutation 4a, the amino acid after mutation of M61 is threonine, the amino acid after mutation of C104 is arginine, the amino acid after mutation of R112 is glutamine, the amino acid after mutation of G181 is alanine, the amino acid after mutation of V189 is isoleucine, and the amino acid after mutation of I327 is valine; and
in the mutation 5a, the amino acid after mutation of L178 is proline.

4. The nitro compound detection element according to any one of claims 1 to 3, wherein the nitro compound is a compound with a structure such that a nitro group is directly linked to a benzene ring and/or a compound having two or more nitro groups.

5. The nitro compound detection element according to any one of claims 1 to 4,
wherein the olfactory receptor protein contains amino acid sequence B composed of amino acid sequence A containing a mutation,
the amino acid sequence A is in a corresponding wild-type olfactory receptor protein and represented by formula (1): φ₇X₁X₂Z₁X₃X₄Z₂Z₃φ₆Uφ₅φ₄φ₃φ₂φ₁X₅X₆ wherein X₁ to X₆ and Z₁ to Z₃ are amino acids derived from the amino acid sequence of a wild-type insect olfactory receptor, φ₁ represents a hydrophobic amino acid, φ₂ to φ₇ each independently represent an uncharged polar amino acid or a hydrophobic amino acid, and U represents an uncharged polar amino acid, and
the olfactory receptor protein satisfies at least one condition selected from the group consisting of the following conditions 1 to 3:
condition 1: in the amino acid sequence B,
X₃ and/or X₄ is substituted, and
X₃ is a branched-chain amino acid and/or X₄ is an uncharged polar amino acid,
condition 2: if X₅ and X₆ in the amino acid sequence A are both a positively charged polar amino acid, X₆ in the amino acid sequence B is an amino acid other than a positively charged polar amino acid, and
condition 3:
if either X₁ or X₂ or both in the amino acid sequence A are a positively charged polar amino acid,
in the amino acid sequence B,
X₁ and/or X₂ is substituted, and
X₁ is an uncharged polar amino acid and/or X₂ is a hydrophobic amino acid.

6. An olfactory receptor protein comprising
(1) amino acid sequence 1C composed of amino acid sequence 1A of SEQ ID NO: 1 or amino acid sequence 1B with at least 80% identity to the amino acid sequence 1A, the amino acid sequence 1A and the amino acid sequence 1B each containing a mutation, wherein the amino acid sequence 1C contains mutation 1a, and the mutation 1a is deletion of a partial or entire region of the first extracellular loop;
(2) amino acid sequence 2C composed of amino acid sequence 2A of SEQ ID NO: 2 or amino acid sequence 2B with at least 80% identity to the amino acid sequence 2A, the amino acid sequence 2A and the amino acid sequence 2B each containing a mutation,
wherein the amino acid sequence 2C contains mutation 2a, and the mutation 2a is substitution 2ax or substitution 2ay,
the substitution 2ax is substitution of at least one amino acid selected from the group consisting of L60, M138, and A152 in the amino acid sequence 2A, and
the substitution 2ay corresponds to the substitution 2ax in the amino acid sequence 2B;
(3) amino acid sequence 3C composed of amino acid sequence 3A of SEQ ID NO: 3 or amino acid sequence 3B with at least 80% identity to the amino acid sequence 3A, the amino acid sequence 3A and the amino acid sequence 3B each containing a mutation,
wherein the amino acid sequence 3C contains mutation 3a and mutation 3b,
the mutation 3a is substitution 3ax or substitution 3ay,
the substitution 3ax is substitution of amino acids S114, A120, H162, V186, V352, and S368 in the amino acid sequence 3A, and the substitution 3ay corresponds to the substitution 3ax in the amino acid sequence 3B,
the mutation 3b is substitution of a charged amino acid into an uncharged amino acid in a region from the third extracellular loop to the third intracellular loop; or
(4) amino acid sequence 4C composed of amino acid sequence 4A of SEQ ID NO: 4 or amino acid sequence 4B with at least 80% identity to the amino acid sequence 4A, the amino acid sequence 4A and the amino acid sequence 4B each containing a mutation,
wherein the amino acid sequence 4C contains mutation 4a, and the mutation 4a is substitution 4ax or substitution 4ay,
the substitution 4ax is substitution of amino acids M61, C104, R112, G181, V189, and I327 in the amino acid sequence 4A, and the substitution 4ay corresponds to the substitution 4ax in the amino acid sequence 4B.

7. A polynucleotide comprising a coding sequence of the olfactory receptor protein of claim 6.

8. A cell comprising the polynucleotide of claim 7.

9. A non-human animal comprising the cell according to claim 8.

10. A nitro compound detection sensor comprising a lipid bilayer, a cell, or a non-human animal containing the cell, the lipid bilayer and the cell each containing the nitro compound detection element of any one of claims 1 to 5.
